# EUROPEAN PATENT APPLICATION

(11) **EP 4 821 482 A1**
(43) Date of publication of application: **14.10.2026**
(21) Application number: 25382350.4
(22) Date of filing: 07.04.2025
(51) Int. Cl.: A61K 9/107, A61K 47/14, A61K 47/18, A61K 47/22, A61K 9/51

(54) **NANO-EMULSIONS AND FORMULATIONS THEREOF FOR DELIVERING ACTIVE INGREDIENTS**

(71) Applicant: Universidade de Santiago de Compostela, 15782 Santiago de Compostela (ES)
(72) Inventor: Alonso Fernandez, Maria Jose, Santiago de Compostela (ES); Lapuhs, Philipp, Santiago de Compostela (DE)
(74) Representative: Hoffmann Eitle

(57) **Abstract**

The present disclosure relates to compositions that include oily nanocores containing oligo/polynucleotides, e.g., in the form of nano-emulsions, for the prevention or treatment of diseases. In particular, it relates to an oil-in-water nano-emulsion composition comprising : (a) an ionizable cationic lipid, (b) a permanently cationic lipid, (c) an oil, (d) a surfactant, (e) an active ingredient comprising an oligonucleotide and/or a polynucleotide, (f) an aqueous medium, optionally comprising , at least one of (g) a PEG-lipid, (h) an amphiphilic polymer, and (i) a hydrophilic polymer, and, further optionally comprising, (j) an additional lipid selected from a phospholipid, a fatty acid, a fatty acid derivative, and a fatty amine, wherein the nano-emulsion composition comprises from 8.0 to 60.0 mol% of ionizable cationic lipid (a), from 8.0 to 40.0 mol% of permanently cationic lipid (b), and a molar ratio of (a) to (b) from 1:1 to 3:1, wherein the mol % is with respect to the total mols of (a), (b), (c), (d), and, if present, (g) and (j).

The present disclosure further relates to said composition in lyophilized form, a reconstructed nano-emulsion from the lyophilized composition, formulations comprising said compositions and a pharmaceutically acceptable excipient, to methods for the preparation of said compositions, and to medical uses of said compositions and formulations for the treatment or prevention of a disease or disorder.

## Description

### TECHNICAL FIELD

The disclosure relates to biology, chemistry and medicine, and more specifically it relates compositions that include oily nanocores containing oligo/polynucleotides, e.g., in the form of nano-emulsions, for the prevention or treatment of diseases.

### BACKGROUND

The delivery of active ingredients, including therapeutic agents, into cells of the body can be accomplished via several different modalities. One such modality is a nanoparticle (NP), also referred to as a nanocarrier, which can be a natural or synthetic material or a combination thereof having a particle size from about 1 nm to about 100 nm and which can access the inside of cells and release its contents.

NPs have been developed for targeting particular cells, tissues or organs. For example, Intl. Patent Application Nos. WO 2019/086627 and WO 2020/221849 describe NPs for systemically targeting cancers and tumors. Some NPs have even been developed for targeted delivery of a therapeutic agent to cells and tissues of the nervous system. See, e.g., Borrajo & Alonso (2022) Drug Deliv. Transl. Res. 12:862-880 and Pinheiro et al. (2021) Int. J. Mol. Sci. 22:11654; see also, Intl. Patent Application Publication Nos. 2013/134777, 2014/12525 and 2015/06146.

The adequacy of particles, and NPs in particular, for drug delivery depends on properties such as their size, surface charge, surface composition and morphology. The intrinsic characterization parameters of NPs, including the determination of size, zeta potential and encapsulation efficiency (EE), and surface composition thus play a role in their biological effects and ultimately their efficacy and safety. Unfortunately, although progress has been made to try to gain access to specific cells tissues or organs and at the same providing a successful delivery of a therapeutic agents, and RNA in particular, with high diffusion, high internalization and high transfection.

Treatments could benefit from, e.g., improvements in the loading of active ingredients in delivery systems, to reduce dosing whilst maintaining good target delivery.

Also, delivery systems should be adapted to the active ingredients they carry. For instance, therapeutic nucleotides, including oligonucleotide- and polynucleotide-based agents, offer promising alternatives to conventional therapies. Therapeutic nucleotides have the potential to address the root causes of such diseases and disorders rather than merely alleviating symptoms. However, their clinical application faces challenges, primarily related to a successful delivery, but also the necessity for less invasive administration techniques.

Thus, the development of suitable delivery systems is crucial to protect and ensure the effective delivery of therapeutic nucleotides to the targeted areas, preferably suitable for various modalities of administration, including, e.g,, intracisterna magna (ICM), intracerebroventricular (ICV), intrastriatal (IST), intratumoral (IT) and intrathecal (ITH) injection and nose-to-brain (N-to-B) delivery, all of them leading to brian delivery as well as other modalities of systemic administration, i.e. intravenous, subcutaneous or intratumoral administration.

### SUMMARY

New delivery systems, nano-emulsions in particular, have been developed for the delivery of active ingredients including therapeutic agents, such as therapeutic nucleotides, e.g., selected from oligonucleotides and polynucleotides, such as RNAs, with improved properties, e.g., enhanced transfection efficiency and lack of any detectable toxicity.

The present disclosure thus relates to such delivery systems. In particular, it relates to an oil-in-water nano-emulsion composition comprising 1. An oil-in-water nano-emulsion composition comprising:
(a) an ionizable cationic lipid,
(b) a permanently cationic lipid,
(c) an oil,
(d) a surfactant,
(e) an active ingredient comprising an oligonucleotide and/or a polynucleotide,
(f) an aqueous medium,
   optionally comprising at least one of
(g) a PEG-lipid,
(h) an amphiphilic polymer, and
(i) a hydrophilic polymer,
   and, further optionally comprising
(j) an additional lipid selected from
   ∘ a phospholipid,
   ∘ a fatty acid,
   ∘ a fatty acid derivative, and
   ∘ a fatty amine,

wherein the nano-emulsion composition comprises
   ∘ from 8.0 to 60.0 mol% of ionizable cationic lipid (a);
   ∘ from 8.0 to 40.0 mol% of permanently cationic lipid (b); and
   ∘ a molar ratio of (a) to (b) from 1:1 to 3:1,
wherein the mol % is with respect to the total mols of (a), (b), (c), (d), and, if present, (g) and (j).

The present disclosure further relates to said composition in lyophilized form, a reconstructed nano-emulsion composition from the lyophilized composition, formulations comprising said compositions and a pharmaceutically acceptable excipient, to methods for the preparation of said compositions and formulations, and to medical uses of said compositions and formulations for the treatment or prevention of a disease or disorder, such as CNS-associated diseases or disorders, PNS-associated diseases or disorders, autoimmune diseases or disorders, liver-associated diseases or disorders, lung-associated diseases, or disorders, spleen-associated diseases or disorders, immunological diseases or disorders, cancers and infectious diseases or disorders. In particular, the present disclosure relates to the nano-emulsion composition or formulation comprising the same for the treatment or prevention of the disease or disorder, and to methods of treatment comprising administering said nano-emulsion composition or formulation comprising the same to a subject suffering from the disease or disorder or methods of preventive treatment comprising to administering said nano-emulsion composition to a healthy subject or to a subject susceptible of contracting a disease or disorder.

The advantages, effects, features and objects other than those set forth above will become more readily apparent when consideration is given to the detailed description below. Such detailed description further refers to the following drawings.

### BRIEF DESCRIPTION OF THE DRAWINGS

To complete the description and to provide for a better understanding of the disclosure, a set of drawings is provided. Said drawings form an integral part of the description and illustrate the disclosure, without being limited thereto or thereby. The drawings comprise the following figures:

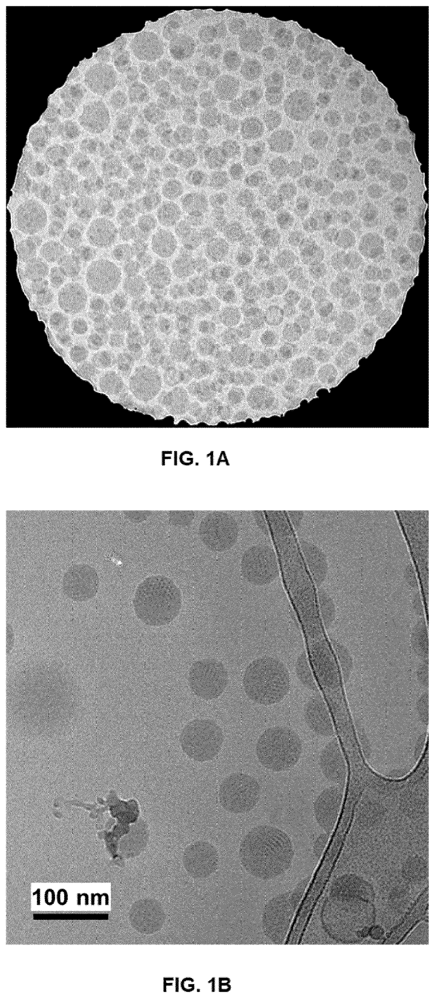

Figure 1 shows cryoTEM images for NEs containing mRNA (Figure 1A) or siRNA (Figure 1B) prepared according to Example 1.

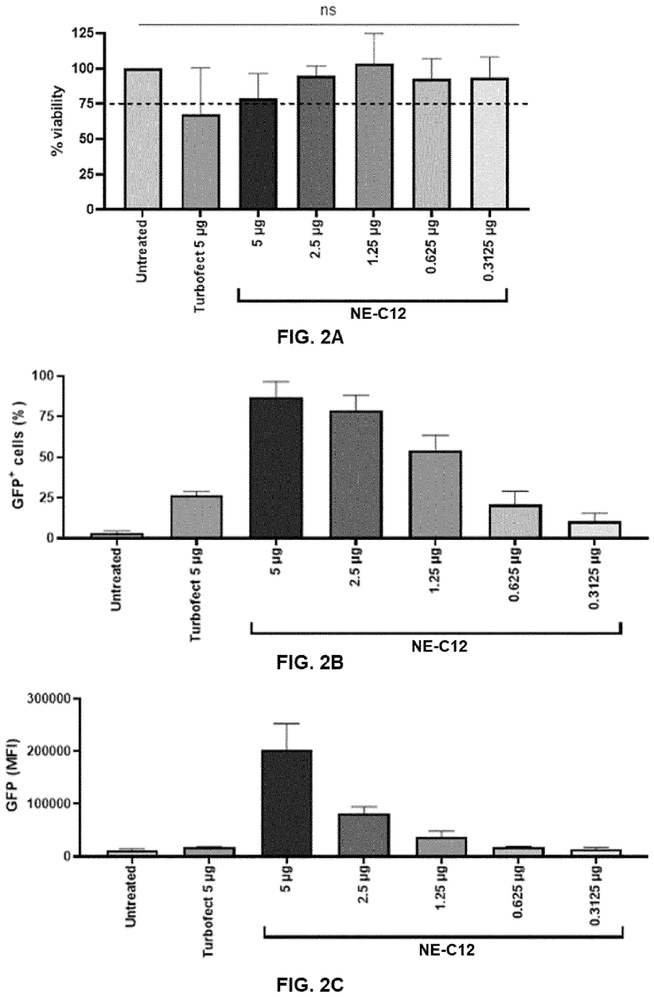

Figure 2 illustrates the *in vitro* performance of NE1 (NE-C12) encapsulating mRNA-GFP as tested in Example 2 including: the *in vitro* toxicity evaluation for SH-SY5Y neurons expressed in terms of % viability (Figure 2A); the transfection efficiency in SH-SY5Y neurons expressed as the percentage of GFP positive (GFP+) cells (%) (Figure 2B); and the transfection efficiency in SH-SY5Y neurons expressed as the mean fluorescence intensity (MFI) of GFP (Figure 2C).

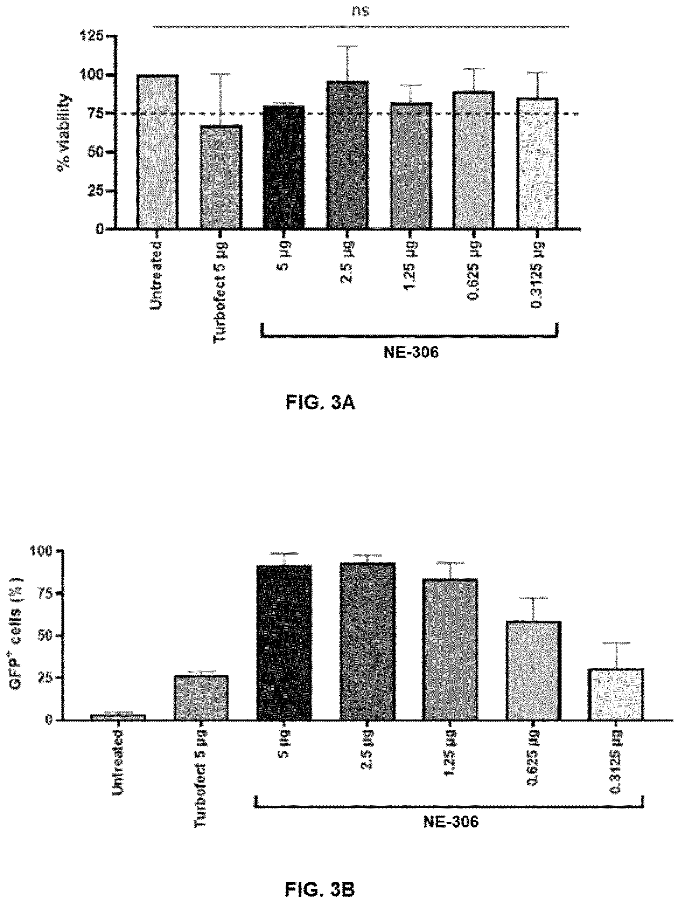

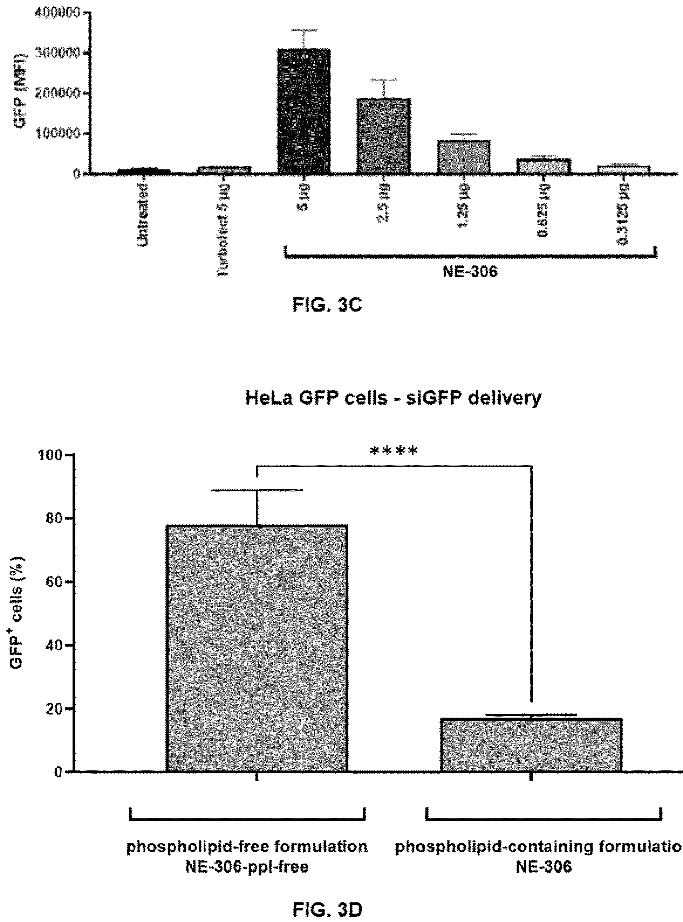

Figure 3 illustrates the *in vitro* performance of NE2 (NE-306) as tested in Example 2 including: the *in vitro* toxicity evaluation for SH-SY5Y neurons expressed in terms of % viability (Figure 3A); the transfection efficiency in SH-SY5Y neurons of mRNA GFP expressed as the percentage of GFP positive (GFP+) cells (%) (Figure 3B); the transfection efficiency of mRNA expressed as the mean fluorescence intensity (MFI) of GFP (for NE-306 (Figure 3C); and the transfection efficiency of siRNA GFP expressed as the percentage of GFP positive (GFP+) cells (%) for NE-306 in comparison with NE-306-ppl-free (NE4) (Figure 3D).

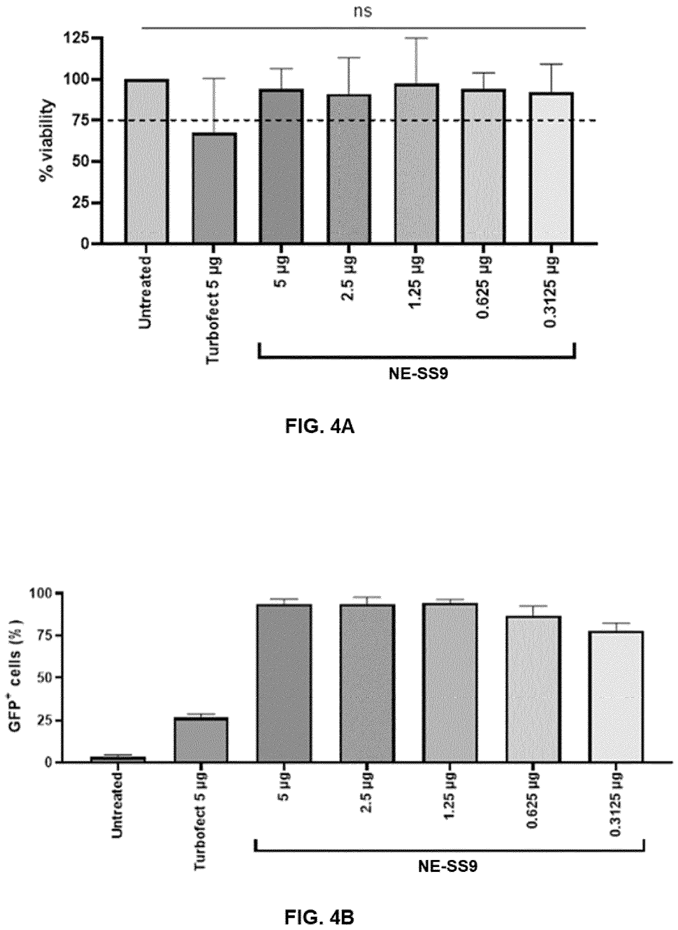

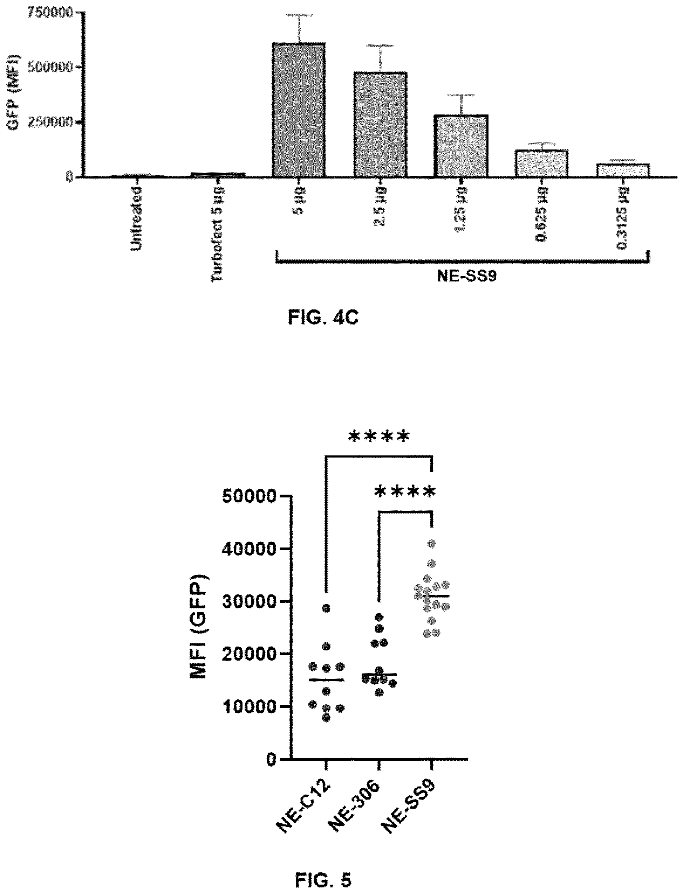

Figure 4 illustrates the *in vitro* performance of NE3 (NE-SS9) as tested in Example 2 including: the *in vitro* toxicity evaluation for SH-SY5Y neurons expressed in terms of % viability (Figure 4A); the transfection efficiency in SH-SY5Y neurons expressed as the percentage of GFP positive (GFP+) cells (%) (Figure 4B); and the transfection efficiency in SH-SY5Y neurons expressed as the mean fluorescence intensity (MFI) of GFP (Figure 4C).
Figure 5 illustrates the *in vivo* performance nano-emulsion compositions in the delivery of mRNA encoding GFP, in terms of the transfection efficiency as evaluated in Example 3A, expressed as mean fluorescence intensity (MFI) of GFP for NE1 (NE-C12), NE2 (NE-306), and NE3 (NE-SS9).

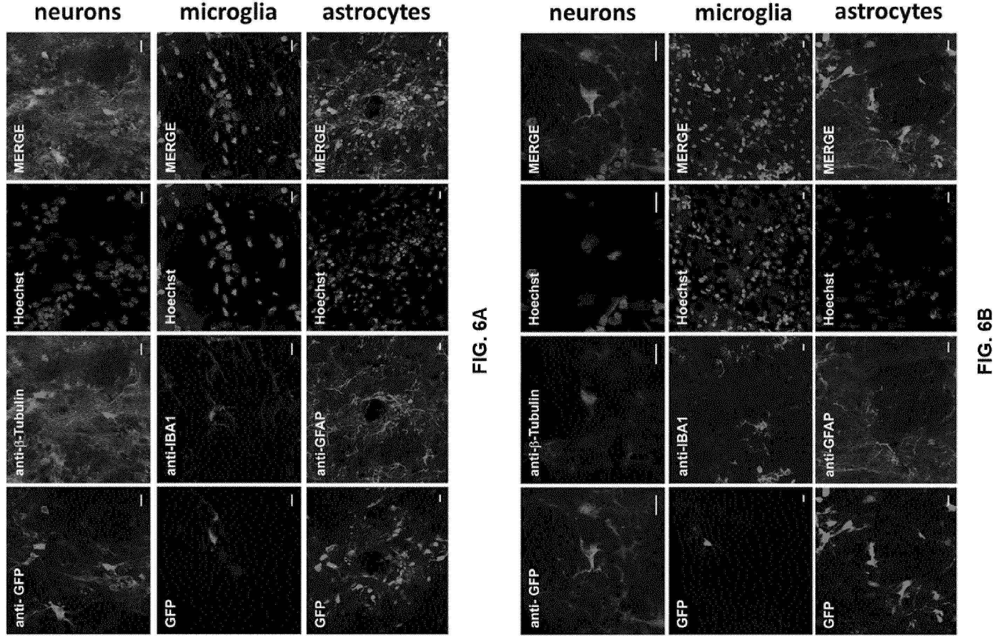

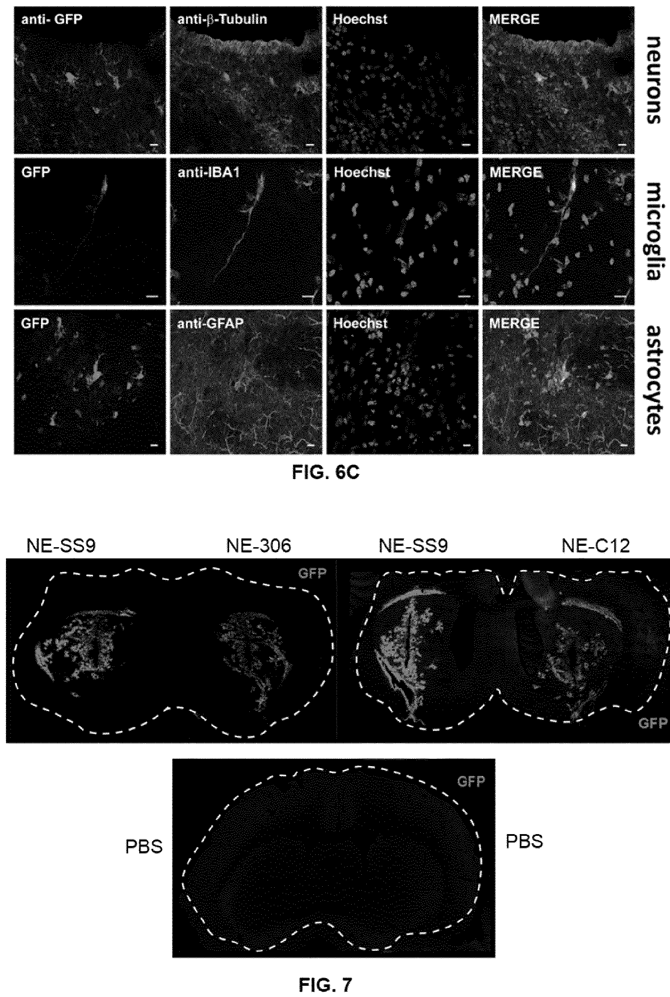

Figure 6 illustrates the *in vivo* performance of nano-emulsion compositions in the delivery of mRNA encoding GFP according to Example 3A, showing images of the *in vivo* colocalization GFP signal induced by NE1 (NE-C12, Figure 6A), NE2 (NE-C306, Figure 6B), and NE3 (NE-SS9, Figure 6C) with specific markers for neurons (including anti-GFP, anti-β-Tubulin and Hoechst), microglia (including GFP, anti-IBA1 and Hoechst), and astrocytes (including GFP, anti-GFAP and Hoechst).
Figure 7 illustrates the *in vivo* performance of nano-emulsion compositions in the delivery of mRNA encoding GFP showing the localization of GFP upon intrastriatal administration of the NEs encapsulating mGFP in a brain mapping of GFP on the coronal plane according to Example 3A, with images at the top from two specimens injected with NE-SS9 (NE3) on the left hemisphere of the brain, the top left image of the specimen further injected with NE-306 (NE2) on the right hemisphere, and the top right image of the specimen further injected with NE-C12 (NE1) on the right globe, and an image at the bottom of an specimen injected with phosphate buffer saline (PBS) on both hemispheres.

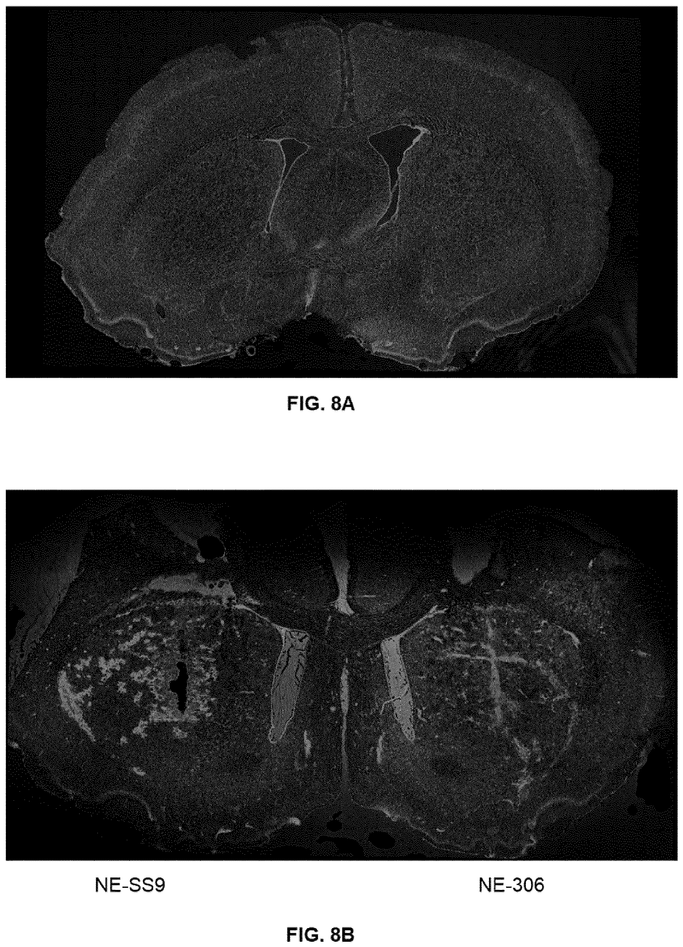

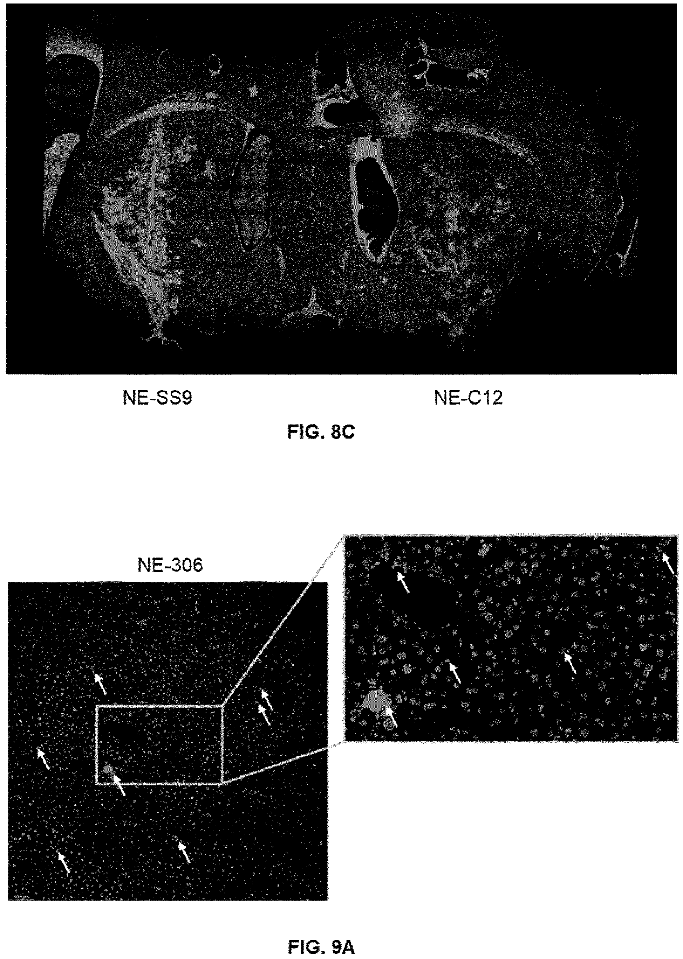

Figure 8 illustrates the *in vivo* performance of nano-emulsion compositions in the delivery of mRNA encoding GFP upon intrastriatal administration according to Example 3A, showing the lack of fluorescence due to lacking GFP expression upon administration of PBS (Figure 8A) and the fluorescence generated by the administration of NEs encapsulating GFP-mRNA (NE-SS9 on the left hemisphere and NE-306 on the right hemisphere (Figure 8B), and NE-SS9 on the left hemisphere and NE-C12 on the right hemisphere (Figure 8C) in the brain.

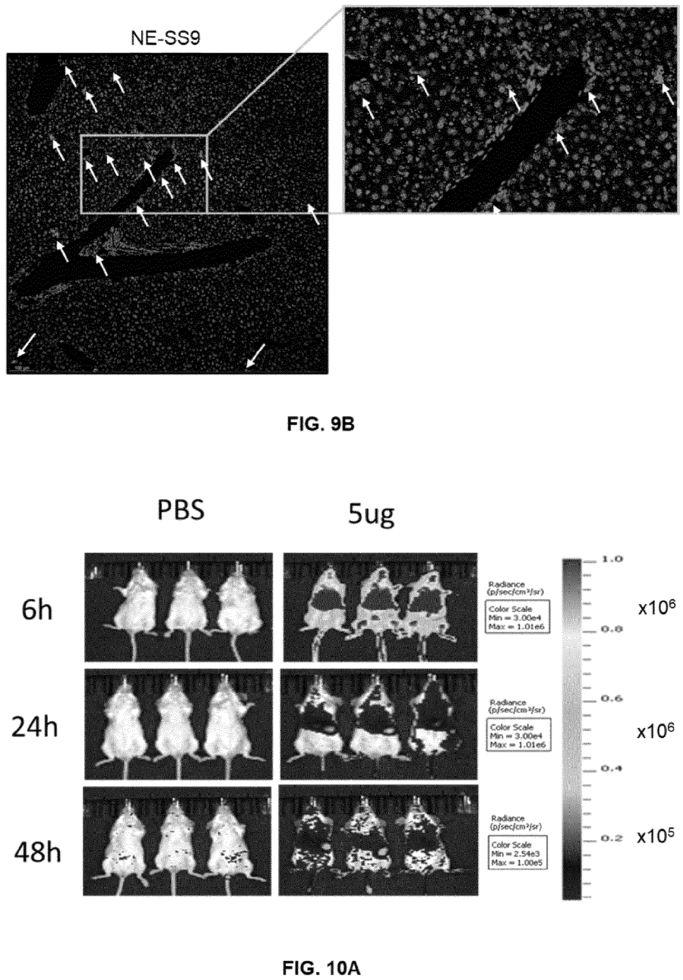

Figure 9 illustrates the *in vivo* performance of the compositions of nano-emulsions (NEs) upon injection of a suspension of NE2 (NE-306, Figure 9A) and of NE3 (NE-SS9, Figure 9B) into a vein of a mouse tail according to Example 3B, showing images of the GFP expression in liver tissue visualized by fluorescence.

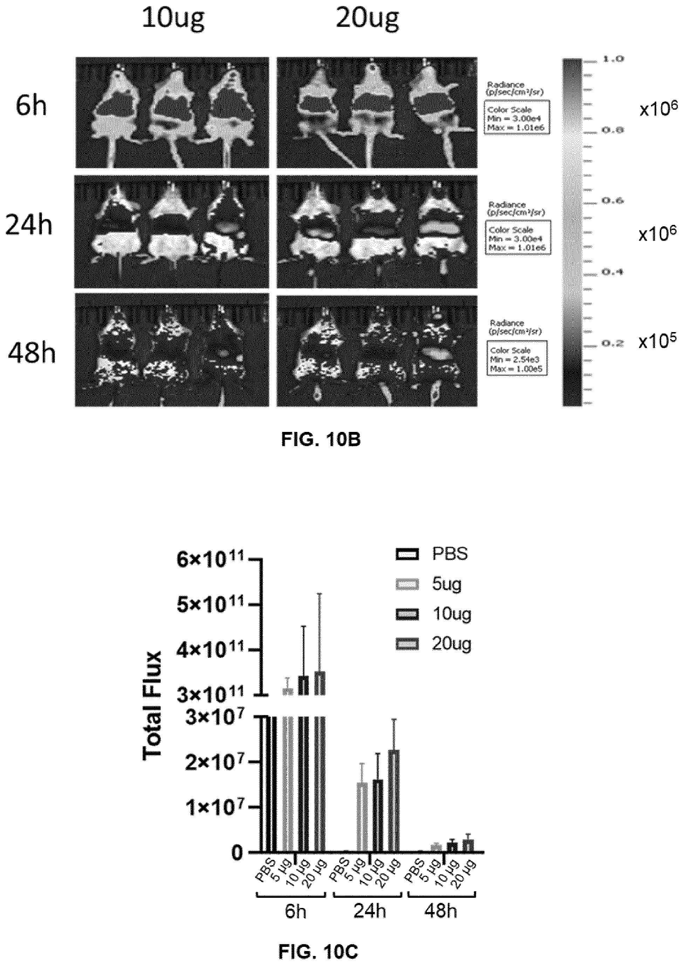

Figure 10 shows the results of in vivo performance of NEs (NE-SS9) loaded with mRNA expressing luciferase (mLuc) after injection into a vein of a mouse tail according to Example 3C, including images of the whole body distribution of Luciferase transfection (Figure 10A for blank with an injection of PBS and an injection of 5 µg of mLuc and Figure 10B for an injection of 10 µg of mLuc and an injection of 20 µg of mLuc) as well as dose-dependency graph in total flux (Figure 10C).

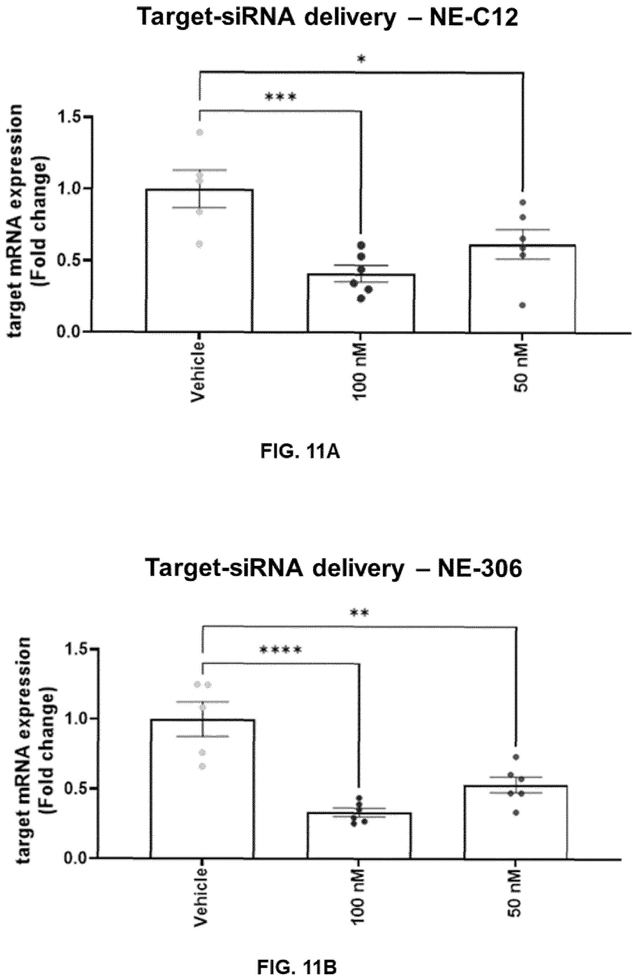

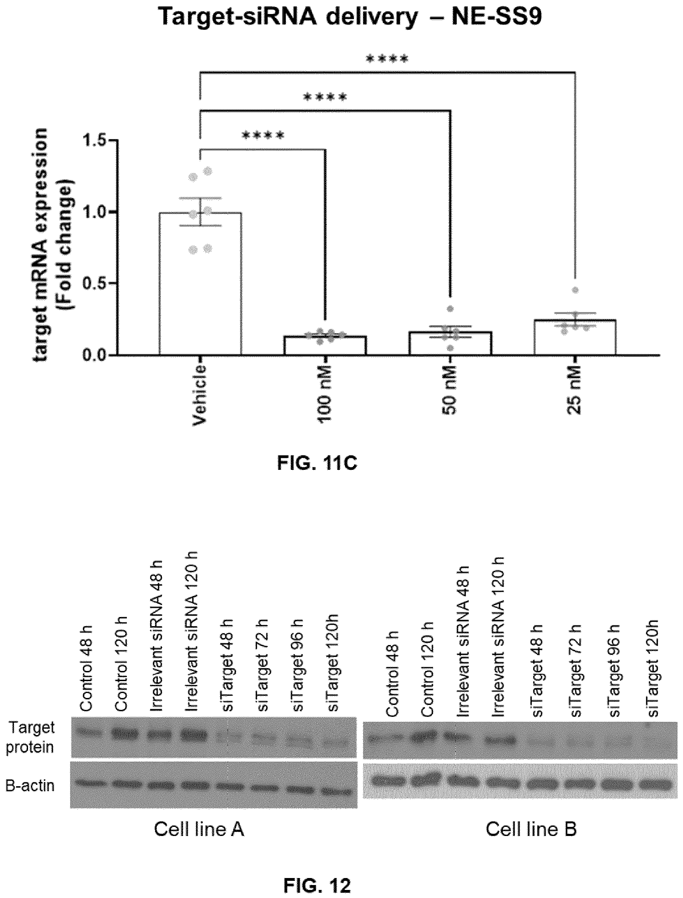

Figure 11 illustrates the *in vitro* performance of the NEs 1-3 comprising a siRNA according to Example 4 showing the fold change on the expression of the target mRNA upon contacting LX-2 hepatic stellate cells with a suspension of the NE-C12 (NE1) in Figure 11A, NE-306 (NE2) in Figure 11B, and NE-SS9 (NE3) in Figure 11C.
Figure 12 illustrates the *in vitro* performance according to Example 4 of NE3 comprising a siRNA showing the results of NE-SS9 delivery of the siRNA on target protein levels in glioblastoma cells: CNS-relevant cell line A (U87) (left) and cell line B (U373) (right).

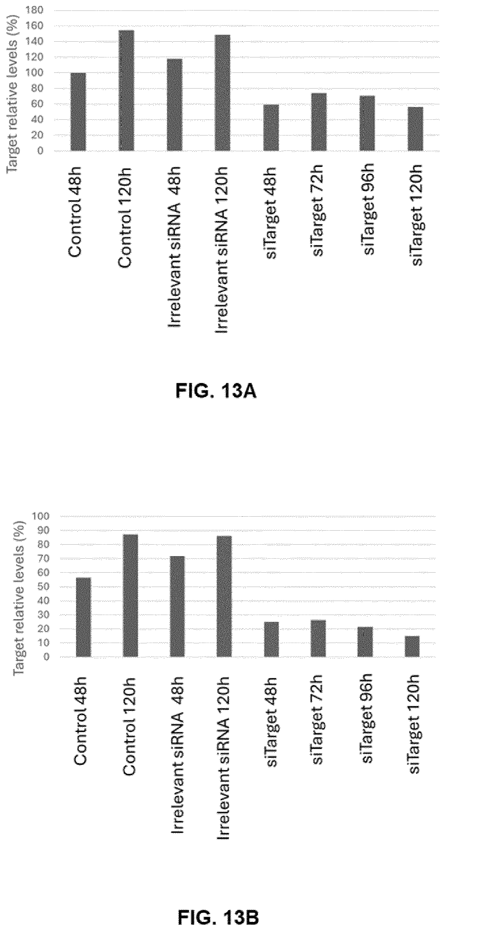

Figure 13 illustrates the *in vitro* performance according to Example 4 of NE3 (NE-SS9) comprising a siRNA showing the kinetic study on the duration of the target knockdown after single treatment with NE-SS9 encapsulating the siRNA for glioblastoma cells: CNS-relevant cell line A (Figure 13A) and cell line B (Figure 13B).

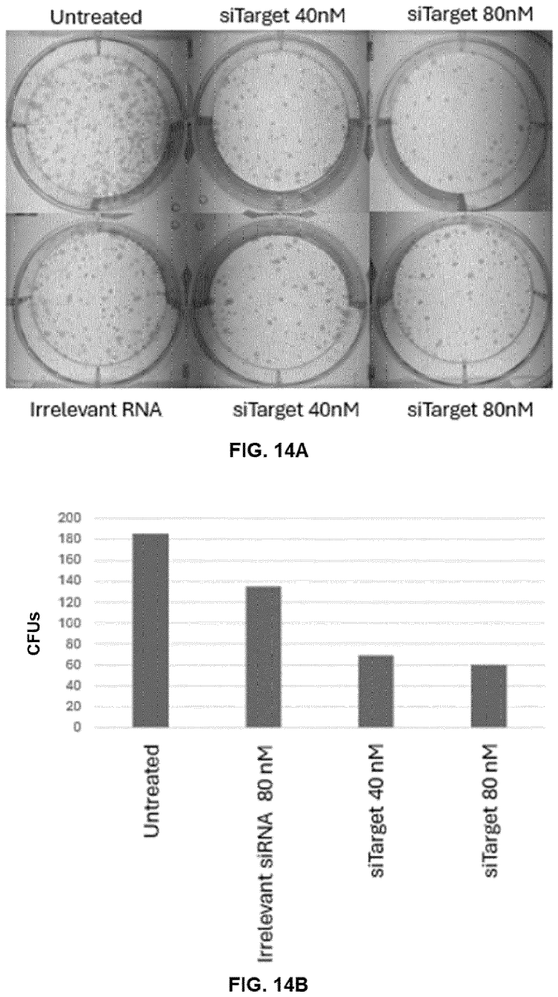

Figure 14 illustrates the *in vitro* performance according to Example 4 of NE3 (NE-SS9) comprising a siRNA in a clonogenic assay upon treatment of glioblastoma cells of CNS-relevant cell line B with NE-SS9 encapsulating the siRNA, alongside untreated cells and cells encapsulating irrelevant siRNA as controls showing images of wells containing viable cells (Figure 14A) and the results in a bar graph, with the number of colony forming units (CFUs) for each treatment (in Figure 14B).

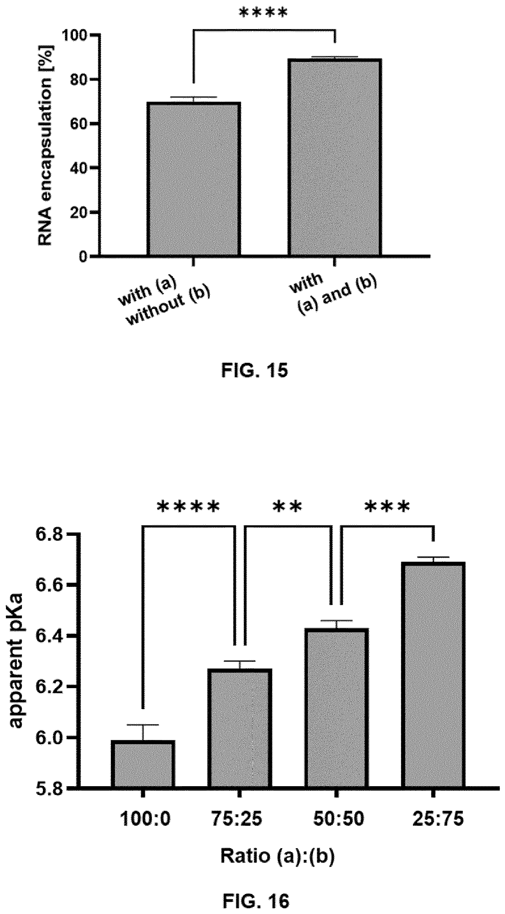

Figure 15 illustrates the RNA encapsulation (%) for NEs according to Example 5, showing an enhanced RNA encapsulation upon use of the combination of an ionizable cationic lipid (a) and a permanently cationic lipid (b) of NE1 (with (a) and (b) combination, right bar) compared to RNA encapsulation using the ionizable cationic lipid (a) without the permanently cationic lipid (b) (with (a) without (b), left bar).
Figure 16 Illustrates the apparent pKa of NEs having varying ratios (a:b) of ionizable cationic lipid (a) and permanently cationic lipid (b), starting with a NE having only ionizable cationic lipid (a) (with a a:b ratio of 100:0), and increasing ratios of permanently cationic lipid (b) (with a:b ratios of 75:25, 50:50 and 25:75) according to Example 6.

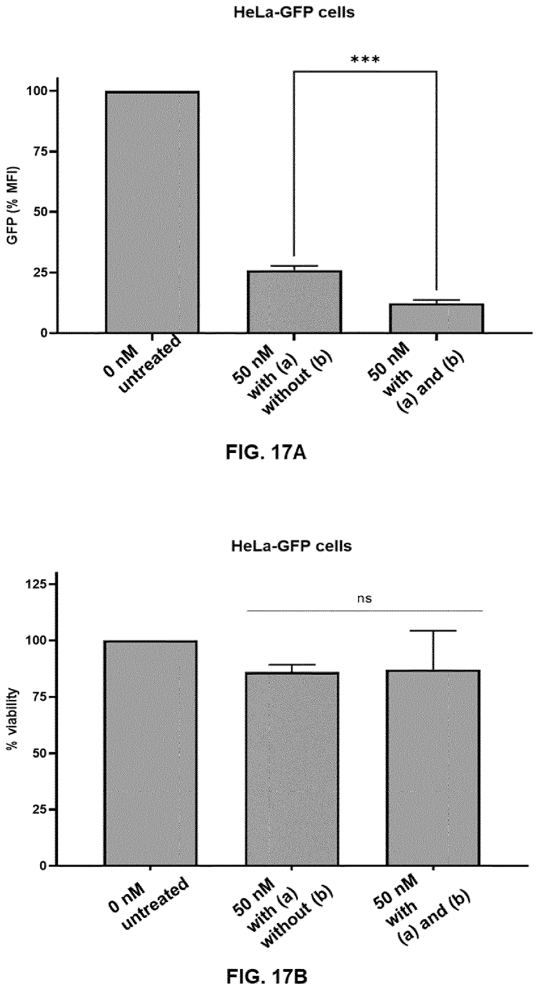

Figure 17 illustrates the transfection efficiency of NEs on HeLa-GFP cells (Figure 17A) and the cell viability of HeLa-GFP cells (Figure 17B) subjected to a treatment with NEs (with and without the combination of an ionizable cationic lipid (a) and a permanently cationic lipid (b)) according to Example 7 (with (a) and (b), right bar, and with (a) without (b) middle bar).

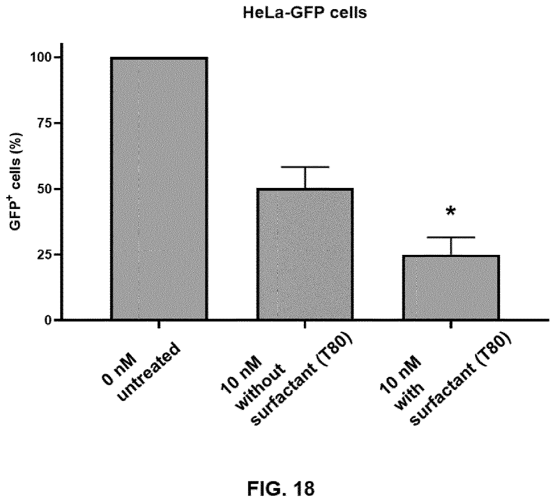

Figure 18 illustrates the transfection efficiency of NEs on HeLa-GFP cells subjected to a treatment with NEs with and without a surfactant according to Example 8.

### DETAILED DESCRIPTION

The present disclosure relates to an oil-in-water nano-emulsion composition comprising:
(a) an ionizable cationic lipid,
(b) a permanently cationic lipid,
(c) an oil,
(d) a surfactant,
(e) an active ingredient comprising an oligonucleotide and/or a polynucleotide,
(f) an aqueous medium,
   optionally comprising at least one of
(g) a PEG-lipid,
(h) an amphiphilic polymer, and
(i) a hydrophilic polymer,
   and, further optionally comprising
(j) an additional lipid selected from
   ∘ a phospholipid,
   ∘ a fatty acid,
   ∘ a fatty acid derivative, and
   ∘ a fatty amine,

wherein the nano-emulsion composition comprises
   ∘ from 8.0 to 60.0 mol% of ionizable cationic lipid (a),
   ∘ from 8.0 to 40.0 mol% of permanently cationic lipid (b), and
   ∘ a molar ratio of (a) to (b) from 1:1 to 3:1,
wherein the mol % is with respect to the total mols of (a), (b), (c), (d), and, if present, (g) and (j).

It has been found that the use of both (a) an ionizable cationic lipid and (b) a permanently cationic lipid favors the encapsulation of active ingredients such as oligo- and polynucleotides (e.g., RNA) and their efficient delivery, e.g., at the intracellular level.

A "nano-emulsion" ("NE") as used herein has the meaning known in the art and may be defined as a fluid colloid system in which liquid droplets and/or liquid crystals are dispersed in a liquid (e.g. oily droplets dispersed in an aqueous medium), wherein the droplets are in then nano-scale in size, e.g., have sizes from 1 nm to 300 nm. An emulsion may be denoted as O/W (for oil in water emulsion) if the continuous phase is an aqueous solution and as W/O (for water in oil emulsion) if the continuous phase is an organic liquid (an 'oil'). Other types of emulsions, such as O/W/O (i.e. oil droplets contained within aqueous droplets dispersed in a continuous oil phase) or W/O/W, are also possible. Emulsions as described herein comprise an oil phase dispersed in an aqueous medium and the oil phase may typically be in the form of droplets dispersed in the aqueous medium, also referred to as oily droplets or oily nanocores. Accordingly, NEs as described herein may be regarded as O/W emulsions where the oily droplets have a nanometer size.

As used herein, "ionizable cationic lipid" means a lipid having a pH-sensitive, polar head group that is neutral at physiological pH but that typically is positively charged at lower pH (e.g., below pH 6).

As used herein, "permanently cationic lipid" means a lipid having a permanently charged positive head group, in other words for which its charged state does not change with the pH.

In several embodiments
- the ionizable cationic lipid (a) is selected from 306Oi10 (tetrakis(8-methylnonyl) 3,3',3",3‴-(((methylazanediyl)*bis*(propane-3,1-diyl))*bis*(azanetriyl))tetra-propionate, CAS#: 2322290-93-5 also referred to herein as 306); SS-OP (bis[2-(4-{2-[4-(cis-9-octadecenoyloxy)phenylacetoxy]ethyl}piperidinyl)ethyl] disulfide, CAS#: 2377474-67-2 also referred to herein as SS9); C12-200 (1,1-((2-(4-(2-((2-(bis(2-hydroxydodecyl)amino)ethyl)(2-hydroxydodecyl)amino)ethyl)piperazin-1-yl)ethyl)azane-diyl)bis(dodecan-2-ol), CAS#: 1220890-25-4, also referred to herein as C12); 1,2-dioleoyl-3-dimethylammonium-propane (DODAP); D-Lin-MC3-DMA ((6Z,9Z,28Z,31Z)-hepta-triaconta-6,9,28,31-tetraen-19-yl 4-(dimethylamino)butanoate, CAS#: 1224606-06-7 also referred to herein as MC3); or cKK-E12 (3,6-bis[4-[bis(2-hydroxydodecyl)amino]butyl]-2,5-piperazinedione, CAS#: 1432494-65-9, also referred to herein as E12), the ionizable cationic lipid may be preferably selected from 306, SS9, and C12, more preferably may be SS9, 306 or C12, and most preferably may be 306 or SS9; and/or
- the permanently cationic lipid (b) is selected from 1,2-dioleoyl-3-trimethylammonium propane (DOTAP), DOTAP variants such as (N)-[1-(2,3-dioleyloxy)propyl]-N,N,N-trimethylammonium chloride) (DOTMA) and/or (1,2-distearoyl-3-trimethylammonium propane) (DSTAP), and cholesterol-based permanently cationic lipids such as (3β-[N-(N-N-dimethylaminoethane)-carbamoyl]cholesterol) (DC-Chol) and/or (Ethylphosphocholine-cholesterol) (EPC-Chol). The permanently cationic lipid may preferably be DOTAP.

A combination of SS9, 306 or C12, as the ionizable cationic (a) and DOTAP as the permanently cationic lipid (b) may be preferred. In several embodiments, a combination of 306 or SS9 as the ionizable cationic (a) and DOTAP as the permanently cationic lipid (b) is particularly preferred.

In several embodiments, the composition comprises an additional lipid (j).

Such additional lipid is different from any other component that may be present in the composition, and, in particular, is different from (a), (b), (c), (d), (e) and, if present, (g), (h) and (i).

The additional lipid (j) may be
- a phospholipid, such as 1,2-dioleoyl-sn-glycero-3-phosphoethanolamine (DOPE), distearoylphosphatidylcholine (DSPC) or soy L-alpha-phosphatidylcholine (soy PC), and/or 1,2-dioleoyl-sn-glycero-3-phospho-(1'-rac-glycerol) (DOPG);
- a fatty acid such as linoleic acid;
- a fatty acid derivative such as glycerol-monooleate; and/or
- a fatty amine such as oleylamine.

The additional lipid may preferably be a phospholipid.

The phospholipid may be preferably selected from 1,2-dioleoyl-sn-glycero-3-phosphoethanolamine (DOPE), distearoylphosphatidylcholine (DSPC) or soy L-alpha-phosphatidylcholine (soy PC), 1,2-dioleoyl-sn-glycero-3-phospho-(1'-rac-glycerol) (DOPG), more preferably may be selected from DOPE, DSPC, and soy PC and more preferably may be DOPE.

The presence of an additional lipid (j) such as a phospholipid has been found to further contribute to performance enhancement, both in transfection efficiency and nano-emulsion stability in biologically relevant media.

A composition as described herein has a concentration of
- (a) ionizable cationic lipid from 8.0 to 60 mol%, in particular from 10.0 to 40.0 mol%, and more in particular from 10.5 to 35.0 mol%;
- (b) permanently cationic lipid from 8.0 to 40 mol%, in particular 9.0 to 30 mol%, more in particular 10.0 to 20 mol%, and more in particular 10.5 to 15 mol%.

Since an additional lipid (j), such as a phospholipid, may optionally be present, said additional lipid may be present at a concentration from 0 to 70 mol%, in particular 5 to 50 mol%, more in particular 7 to 25 mol%, and yet more in particular 8 to 21 mol%.

Additionally, or alternatively, the total concentration of combined amount of (a), (b) and, if present (j), may be from 21 to 86 mol%. In several embodiments the concentration of the combination of (a), (b) and, if present, (j) is at least 40 mol%, preferably at least 45 mol%, in particular at least 48 mol%, more in particular at least 50 mol%, yet more in particular at least 52 mol%, and even more in particular at least 53 mol%.

The mol% is based on the mols of (a), (b) or (j) with respect to the total mols of (a), (b), (c), (d), and, if present, (g) and (j).

Such mol% have been found to contribute to providing stable and efficient nano-emulsions, compositions outside of these ranges were found to work but less efficiently.

In a NE as described herein the relative molar ratio of (a) ionizable cationic lipid to (b) permanently cationic lipid (a):(b) is from 1:1 to 3:1.

In several particular embodiments, the composition comprises the additional lipid (j), the additional lipid preferably is a phospholipid and the phospholipid preferably is DOPE. The preferences described above apply to this combination. In particular, the NE composition may preferably comprise
(a) SS9, 306 or C12, more preferably SS9 or 306;
(b) DOTAP; and
(j) DOPE.

Compositions as described herein comprise an oil (c). As used herein, "oil" means a non-polar chemical that consists mostly of hydrocarbon. Herein, an oil is a liquid that is insoluble in water and that forms droplets in the presence of water and one or more surfactants. The oil (c) is distinct from any other component in the composition and in particular is different from (a), (b), (d), (e) and, if present, (g), (h), (i) and (j). In several embodiments, compositions as described herein comprise an oil (c) selected Vitamin E (Vit E), oleic acid, ethyl oleate, isopropyl myristate, soybean oil or squalene and squalene derivatives, and a combination of medium-chain triglycerides (TGs), the combination of medium chain TGs preferably comprises caprylic acid and capric acid, and more preferably comprises Labrafac^{™} Lipophile, WL 1349 (WL 1349) or Labrafac^{™} PG (PG). In several particular embodiments the oil (c) is preferably selected from Vit E and oleic acid and may preferably be Vit E.

It has been found that the incorporation of such oils leads to stable nano-emulsion formation. Further, without being bound to any theory, the oil itself may contribute to enhanced fluidity, enabling the consequently less rigid droplets in the nano-emulsions to penetrate deeper into tissues or diffuse better across the brain. Vitamin E, as antioxidant, may additionally protect other components of the nano-emulsion or the cells that engulf the droplets of the nano-emulsions from oxidation.

Compositions as described herein comprise a surfactant (d). As used herein, "surfactant" means a compound or molecule that decreases surface tension or interfacial tension between two liquids, such that it can serve as an interface between aqueous medium and oil / lipid droplets). Surfactants can be divided into four basic types: anionic, cationic, non-ionic and zwitterionic (i.e., amphoteric).

The surfactant (d) is different from other components in the NE composition and in particular is different from (a), (b), (c), (e) and if present (g), (h), (i) and (j).

In several embodiments, in a composition as described herein the surfactant (d) is a non-ionic surfactant.

As used herein, "non-ionic surfactants" are not charged and may typically be saturated or weakly unsaturated hydrocarbon chain substances on which several polar groups are linked, e.g. ethylene or propylene oxides.

The non-ionic surfactant may be preferably selected from surfactants derived from polyethoxylated sugar and fatty acids, such as Tween^{®} 80 (T80) (derived from polyethoxylated sorbitan and oleic acid) and derived from polyglycol and fatty acids (preferably hydroxy fatty acids), such as Kolliphor^{®} HS 15 (HS15) (derived from polyethylene glycol 15 and 12-hydroxystearic acid), TPGS (α-Tocopherol polyethylene glycol succinate), and poloxamers. More preferably the non-ionic surfactant is T80.

In some embodiments, a composition as described herein has
- a concentration of the oil (c) from 10 to 80 mol%, in particular from 20 to 60 mol%, more in particular from 30 to 55 mol%; and/or
- a concentration of the surfactant (d) from 0.1 to 40 mol%, in particular from 1 to 30 mol %, more in particular from 3 to 15 mol%, yet more in particular from 5 to 12 mol%.

The mol% being with respect to the total mols of (a), (b), (c), (d), and, if present, (g) and (j), in the composition.

In some embodiments, a composition as described herein has a concentration of the oil (c) of above 50 mol%, e.g., from 50.25 to 80 mol.%, in particular from 50.5 to 60 mol% and more in particular from 51 to 55 mol%.

In some embodiments, a composition as described herein has a concentration of the surfactant (d) below 2.5 mol%, e.g., from 0.1 to 2.5 mol%, in particular from 0.2 to 2.0 mol%.

Concentrations of the different components as described herein have been found to contribute to the encapsulation of active ingredients (e.g., therapeutic agents) and to the advantageous properties of the composition for the delivery of said active ingredients.

Nano-emulsion compositions as described herein may further comprise at least one of:
(g) a PEG-lipid,
(h) an amphiphilic polymer, and
(i) a hydrophilic polymer.

Such components (g), (h) and (i) are distinct from each other and from other components in the NE composition, in particular are different from (a), (b), (c), (d), (e) and, if present, (j).

It may be preferred for NE compositions as described herein to at least comprise a PEG-lipid (g). In some particularly preferred embodiments NE compositions as described herein comprise a PEG-lipid (g) and do not comprise an amphiphilic polymer (h) or a hydrophilic polymer (i).

In some other embodiments NE compositions as described herein comprise an amphiphilic polymer (h) and/or hydrophilic polymer (i) and do not comprise a PEG-lipid (g).

The PEG-lipid may also be referred to as a PEGylated lipid. PEG-lipids are polyethylene glycol (PEG)-lipid conjugates, i.e., PEG derivatives containing lipid molecules.

The PEG-lipid (g) may be preferably is selected from 1,2-distearoyl-sn-glycero-3-phosphoethanolamine-poly(ethylene glycol) (DSPE-PEG), 1,2-dimyristoyl-sn-glycero-3-methoxypolyethylene glycol (DMG-PEG), and 1,2-distearoyl-rac-glycero-3-methylpolyoxyethylene (DSG-PEG), and most preferably the amphiphilic polymer is DSPE-PEG.

Amphiphilic polymers, may also be referred to as amphipathic polymers, are copolymers containing both hydrophilic and hydrophobic segments.

In a NE composition as described herein the amphiphilic polymer (h) may preferably be a partly negatively charged amphiphilic polymer, more preferably a polyglutamic acid (PGA)-PEG.

Hydrophilic polymers are polymers which have affinity to water, e.g., dissolve in, or are swollen by, water.

In a NE composition as described herein the hydrophilic polymer (i) may preferably be a negatively charged hydrophilic polymer, such as hyaluronic acid (HA) or Dextrane or polysialic acid (PSA) or alginate.

Such PEG-lipids (g), amphiphilic (h) and/or hydrophilic (i) polymers may further contribute to the encapsulation and delivery of the active ingredient.

In some embodiments, a composition as described herein may have a concentration of PEGylated lipid (g) from 0.01 to 15 mol%, in particular from 0.1 to 10 mol % and more in particular from 0.5 to 5 mol%, wherein the mol % is with respect to the total mols of (a), (b), (c), (d), and, if present, (g) and (j).

In some embodiments, a composition as described herein may have a total concentration of amphiphilic polymer (h) and/or hydrophilic polymer (i), from 0.01 to 15 mol%, wherein the mol % is with respect to the total mols of (a), (b), (c), (d), and, if present, (g) and (j).

In some embodiments, the weight ratio of the total weight amphiphilic polymer (h) and/or hydrophilic polymer (i) with respect to the total weight of a), b), c), d), and, if present, g) and j) is from 1:1 to 1:50.

In several embodiments, a nano-emulsion composition as described herein comprises:
(a) 306, SS9 or C12, as the ionizable cationic lipid, preferably 306 or SS9;
(b) DOTAP as the permanently cationic lipid;
(c) Vit E as the oil, and
(d) T80 as the surfactant,
   and preferably also
(g) DSPE-PEG as PEG-lipid, and(j) DOPE as the additional lipid.

It has been found that such combination results in stable, potent and biocompatible nano-emulsions for the delivery of active ingredients (such as nucleotides including RNA).

The oil phase of the nano-emulsions may be in the form of droplets dispersed in the aqueous phase constituted by the aqueous medium. In a nano-emulsion composition as described herein the droplets are in the nano-scale, having lengths in two or three dimensions and having a diameter from about 1 nm to about 300 nm, that can be used to transport, for example, an active ingredient such as a therapeutic agent. Nano-emulsions typically possess unique physical and chemical properties due to their high surface area and nanoscale size of their droplets. Where the NEs include an active ingredient (e.g., a therapeutic agent), they may be referred to as complexed NEs, such as NEs complexed with therapeutic oligo- or polynucleotides such as RNA, also simply referred to as RNA NEs.

The ionizable cationic lipid (a), permanently cationic lipid (b), the oil (c), the surfactant (d), the active ingredient (e) and, if present, the PEG-lipid (g) , the amphiphilic polymer (h) and/or hydrophilic polymer (i), and the additional lipid (j) may form oily nanodroplets containing the active ingredient (e), e.g., an RNA, condensed by (a), (b) and, if present, (j) dispersed in the oil (c). An example of such droplets can be seen in Figure 1. The surfactant (d) is at the surface of the droplets. If present the PEG-lipid (g), amphiphilic polymer (h) and/or the hydrophilic polymer (i), may also be at the surface of the droplets.

In some embodiments, the droplets in the nano-emulsions may be structured. For instance, structures may refer to internal organization of the lipids and RNA complexes within the oily droplets, which may include lamellar, cubic or hexagonal organization. Or, the structure may refer to the overall organization of the nano-emulsions, which may be organized in the following layers: (1) a core and (2) a shell that may or may not be charged.

In some particular embodiments, the droplets in the nano-emulsion form a hydrophobic, oily core in which the active ingredient (e) may be condensed and entrapped by the ionizable lipid (a) and the permanently cationic lipid (b). The active ingredient may also be entrapped within or on top of the surface shell by said ingredients.

A nano-emulsion composition as described herein may further comprise a polymeric coating wherein the polymeric coating is dispersed on surfaces of the droplets of the nano-emulsion.

Depending on the nature of the active ingredients, these can complex within and/or on the surface of the droplets through, for example, electrostatic forces and/or hydrophobic/hydrophilic interactions. Accordingly, the amount of active ingredient in the different phases of the droplets, e.g., in core or within/on the surface of the droplets may vary.

The active ingredient (e) is selected from, e.g., nucleotides (including oligonucleotides and polynucleotides).

In some embodiments, the active ingredient (e) may be charged, preferably may be negatively charged. Preferably the negatively charged compound or molecule may be a therapeutic nucleotide, preferably selected from therapeutic oligonucleotides and polynucleotides such as RNA. Without being bound to any theory, it is believed that negatively charged therapeutic agents (such as RNA) are bound/complexed by the cationic and ionizable lipids via electrostatic interaction, the formation of such a complex may result in a somewhat "net-neutral" structure.

In some embodiments the active ingredient (e) may be a therapeutic oligonucleotide, yet more preferably the therapeutic oligonucleotide may be a single stranded (ss) and/or double stranded (ds) therapeutic oligonucleotide.

As used herein, "polynucleotide" or "nucleic acid" means a polymer of nucleotides. Although it may comprise any type of nucleotide units, the term generally applies to nucleotide polymers of DNA, cDNA and/or RNA. Polynucleotide is used to include single-stranded (ss) nucleic acids, double-stranded (ds) nucleic acids, and DNA, cDNA and/or RNA made from nucleotide or nucleoside analogues that may be identified by their sequences, which are generally presented in the 5' to 3' direction (as the coding strand), where the 5' and 3' indicate the linkages formed between the 5' hydroxyl group of one nucleotide and the 3'-hydroxyl group of the next nucleotide. For a coding strand presented in the 5'-3' direction, its complement (or non-coding strand) is the strand that hybridizes to that sequence according to Watson-Crick base pairing. Thus, as used herein, the complement of a nucleic acid such as a polynucleotide is the same as the "reverse complement" and describes the nucleic acid that in its natural form, would be based paired with the nucleic acid in question, including antisense oligonucleotides (ASOs).

As used herein, "oligonucleotide" means a short nucleic acid compound (e.g., a polymer of less than about 100 nucleotides in length) that may include deoxyribonucleotides (or modified deoxyribonucleotides), ribonucleotides (or modified ribonucleotides) or both. Likewise, an oligonucleotide may be ss or ds and thus may or may not have duplex regions.

As used herein, "therapeutic nucleotide" means a ss or ds nucleic acid that has a therapeutic application (i.e., application in treating a disease, disorder or condition). Such nucleic acids may contain one or more modified nucleotide residues or linkages and also can include a targeting ligand and/or delivery vehicle.

In some particular embodiments, the active ingredient (e) is selected from a RNA-based therapeutic nucleotide, such as a messenger RNA (mRNA)-, a small interfering RNA (siRNA)-, a micro RNA (miRNA)-, a long noncoding RNA (IncRNA)-, a circular RNA (circRNA)-, an antisense oligonucleotide (ASO)-, self-amplifying RNA (saRNA)-, an activating RNA (aRNA)- (including short activating RNAs (saRNAs)), an editing RNA (eRNA)-, an inhibiting RNA (iRNA)-, a single-stranded guide RNA (sgRNA)-, a transfer RNA (tRNA)- (including suppressor tRNAs (sup-tRNAs)-), a decoy RNA-based therapeutic nucleotide and a combination thereof. Preferably, the active ingredient may be selected from a mRNA and a siRNA.

As used herein, "messenger RNA" or "mRNA" means a nucleic acid that includes RNA and that mediates translation of a nucleotide sequence to an amino acid sequence.

As used herein, "editing RNA" or "eRNA" means a nucleic acid that contains RNA and that mediates inserting, deleting and even base substituting of nucleotides within a target nucleotide sequence. RNA editing has been observed in a number of different types of RNA such as, for example, mRNA, microRNA (miRNA), single-stranded guide RNA (sgRNA), transfer RNA (tRNA) and ribosomal RNA (rRNA). RNA editing can be enzymatically mediated either by exogenously supplying adenosine deaminase acting on RNA enzyme (ADAR) or by directing an endogenous ADAR to a specific site in a target RNA nucleotide sequence, and typically involves editing at a single nucleotide site by directing the ADAR to that site through complimentary oligonucleotides. Other RNA editing systems are known such as, for example, CRISPR. eRNAs typically are ss.

As used herein, "inhibiting RNA" or "iRNA" means a nucleic acid that contains RNA and that mediates the targeted cleavage of a RNA transcript via RNA interference, for example, through a RNA-induced silencing complex (RISC) pathway. Some iRNAs are ss and other iRNAs are ds and have a sense strand and an antisense strand, where the sense strand and the antisense strand form a duplex. iRNA directs sequence-specific degradation of mRNA via RNA interference. iRNA attenuates, inhibits, modulates or reduces expression of a target nucleotide sequence in a cell. Examples of iRNA include, but are not limited to, an antisense oligonucleotide (ASO), Dicer substrate interfering RNA (DsiRNA), miRNA, short hairpin RNA (shRNA) and small interfering RNA (siRNA).

As used herein, "single-stranded guide RNA" or "sgRNA" means a nucleic acid that includes RNA and that mediates target-specific cleavage by a nuclease for its DNA sequence site such as, for example, in CRISPR-Cas9 gene editing.

As used herein, "microRNA" or "miRNA" are small, single-stranded, non-coding RNA molecules containing 21-23 nucleotides.

As used herein, "decoy RNA" is a strand of RNA with miRNA-binding domains that sequester the corresponding miRNA into stable complexes through complementary base-pairing, disabling a particular RNA interference pathway.

The active ingredient (e) may be present in any suitable amount, e.g., depending on the amount that can be encapsulated and the necessary amount for a specific formulation or treatment.

As a mode of example, in some embodiments, in a composition as described herein
- the active ingredient (e) is present at a concentration of at least 0.0125 mg/mL with respect to the total volume of the nano-emulsion, preferably from 0.0125 mg/mL to 10.0 mg/mL, and/or
- the weight ratio of active ingredient (e) to the total weight of (a), (b), (c), (d), and, if present, (g) and (j), is from 1:5 to 1:100.

In some embodiments, the active ingredient is present at a concentration of at least 0.025 mg/mL, in particular at least 0.05 mg/mL, more in particular at least 0.10 mg/mL, yet more in particular at least 0.3 mg/mL, with respect to the total volume of the nano-emulsion. It has been found that given the improved encapsulation of active ingredients in the nano-emulsion compositions as described herein, the amount of active ingredient present in the nano-emulsion composition may be increased. In particular, since the combination of lipids (a), (b) and, if present, (j) improves encapsulation efficiency, the relative active ingredient (e.g., RNA) loading (w/w%) can be increased by reducing the weight ratio of other components to the active ingredient.

In some embodiments, the active ingredient is present at a concentration of at most, more in particular at most 0.5 mg/mL, yet more in particular at most 0.25 mg/mL, and even more in particular at most 0.20 mg/mL, with respect to the total volume of the nano-emulsion. In some embodiments, the active ingredient is present at a concentration from 0.10 to 0.20 mg/mL with respect to the total volume of the nano-emulsion.

It has been found that given the improved delivery such low amounts of active ingredient may be suitably used.

In some embodiments, the weight ratio of active ingredient (e) to the total weight of (a), (b), (c), (d), and, if present, (g) and (j), is from 1:7 to 1:50, in particular from 1:10 to 1:35, and more in particular from 1:15 to 1:25, yet more in particular is of about 1:19.

If present, a polymeric coating as described above can be a negatively charged coating such as, for example, a PSA coating or a HA coating or polyglutamic acid coating or a dextran coating or an alginate coating or can be positively charged such as protamine or polyarginine or collagen or chitosan or can be a neutrally charged coating such as, the said polymers linked to PEG moieties or hydrophobic segments linked to PEG. In some instances, the polymeric coating can be present at a weight ratio of 0.05 to 50 relative to the total weight of (a), (b), (c), (d), and, if present, (g) and (j). In some particular embodiments, the amphiphilic polymer (h) and/or the hydrophilic polymer (i) as described above may constitute such a polymeric coating.

The polymeric coating may be dispersed on surfaces of the droplets in the nano-emulsion.

In several embodiments, NE compositions as described herein may have an apparent pKa from 5 to 7. The apparent pKa may be determined by means known in the art. For instance, the TNS (6-(p-Toluidino)-2-naphthalenesulfonic acid) assay may be used. Apparent pKas between 5-7 are found to be below the pH of extracellular fluids and above the pH of inside the endosomes. Without being bound to any theory, NEs having such apparent pKas may contribute to ensure that the droplets of the NEs are neutral in circulation through the body and that they are positively charged within the acidifying endosomes, improving tolerability and efficacy of NEs for the delivery of their cargo. Additionally, the ability to adjust the apparent pKa of the NEs by varying ratios of (a):(b) might allow for cell specific delivery, as differences in intracellular trafficking and endosomal acidification behavior have been described.

In several embodiments, in a composition as described herein the oil phase is in the form of droplets dispersed in the aqueous phase having
(I) an average diameter of ≤ 300 nm, e.g. from 1 to 300 nm; preferably of ≤ 100 nm, e.g., from 5 to 100 nm; more preferably ≤ 80 nm, e.g., from 10 to 80 nm, and yet more preferably ≤ 65 nm, e.g. from 25 to 62 nm;
(II) a Z-potential from -30 mV to +35 mV, preferably from - 10 to + 8 mV, more preferably from - 5 to + 5 mV, and yet more preferably from - 2.0 to + 2 mV;
(III) a polydispersity index (PDI) of below 0.35, e.g., from 0.01 to 0.35, preferably below 0.25, e.g., from 0.05 to 0.25; and/or
(IV) an encapsulation efficiency (EE) of at least 60 %, preferably at least 90% and more preferably at least 95 %, yet more preferably of 100%.

In several embodiments the droplets of the nano-emulsion have (I) as defined above and optionally one or more of (II), (III) and (IV) as also defined above.

The average diameter of the droplets refers to the hydrodynamic diameter and may be expressed in terms of the D50 particle size, which is the median particle diameter corresponding to the 50^{th} percentile of the cumulative undersize distribution. For instance, for a D50 particle size of 250 nm, 50% of the droplets in a sample of the nano-emulsion composition will be larger and 50% of the particles will be smaller. The hydrodynamic diameter is the diameter of a hypothetical hard sphere that diffuses with the same speed as the particle being measured. In practice, droplets are solvated and can be spherical, spherical-like or non-spherical, while moving dynamically in solution. The determined diameter is therefore an indicator of the apparent size of the solvated droplet that is approximated as being spherical.

The polydispersity index also referred to as PDI, is a measure of the heterogeneity of droplet sizes in the nano-emulsion composition. If droplets in the nano-emulsion are uniform, the resulting size distribution will be narrow and the PDI small, determining that the sample is monodisperse.

The average diameter (e.g. the D50) and the PDI may be measured by methods known in the art, e.g., dynamic light scattering (DLS). DLS measures the fluctuations in scattered light intensity with this being correlated to the particle hydrodynamic diameter via the correlation function and the Stokes-Einstein equation. Methods may be used such as those defined in the ISO standards ISO 22412:2017 (Particle size analysis - Dynamic light scattering (DLS)) and ISO 13320-1:2020 (Particle size analysis - Laser diffraction methods), whereby the sample is irradiated by laser light and the particle-size distribution of the sample is determined from the intensity pattern of diffracted and scattered light, and the D50 may be expressed based on a volume distribution. The measurements may be performed, e.g., after diluting samples of the compositions from 5x to 200x, depending on the concentration of the droplets in the NE, for instance about 10 x or about 20x in formulation buffer, e.g. PBS 1x or TBS 1x. An adequate apparatus such as a Zetasizer^{®} Nano ZS (Malvern Instruments) may be used.

Z-potential or ZP refers to zeta (ζ) potential or electrokinetic potential and is a measure of the magnitude of the electrostatic repulsion/attraction between particles, or droplets in the nano-emulsion, and is one of the fundamental parameters known to affect stability of dispersed systems. Z-potential may be measured by means known in the art such as in terms of mean electrophoretic mobility values, e.g., by laser Doppler electrophoresis. Methods may be used such as those defined in the ISO standard ISO 13099-1:2012 (Colloidal systems - Methods for zeta-potential determination Part 1: Electroacoustic and electrokinetic phenomena). The measurements may be performed, e.g., after diluting samples of the compositions from 5x to 200x, depending on the concentration of the droplets in the NE, for instance about 10x or about 20 x in formulation buffer, e.g. PBS 1x or TBS 1x. An adequate apparatus such as a Zetasizer^{®} Nano ZS (Malvern Instruments) may be used.

The encapsulation efficiency (EE) may be expressed in terms of a % and defined as the % of active ingredient (e.g., therapeutic agent) that is encapsulated by the nano-emulsion, e.g., in the droplets. The EE may be determined after preparation of the composition by isolating the capsules and disrupting the capsules in the composition and determining the amount of therapeutic agent after disruption (by treating the capsules with a disrupting agent and analyzing the released amount of polynucleotide by appropriate methods. As a mode of example, where the therapeutic agent is a therapeutic RNA the EE% and the concentration of the RNA agent encapsulated may be determined by using, e.g., a Quant-iT RiboGreen RNA assay kit (Invitrogen).

A nano-emulsion composition as described herein comprises the oily phase dispersed in an aqueous medium, e.g., in the form of droplets dispersed in the aqueous medium.

In some embodiments, the aqueous medium is water, or an aqueous solution selected from a saline, a buffered aqueous solution or a saline buffered aqueous solution. Saline is a sodium chloride aqueous solution. The buffered aqueous solution comprises water or saline and a buffering agent, preferably the aqueous solution may be a saline-containing buffered solution also referred to as buffer saline. A buffer saline may be ., e.g., selected from a phosphate buffer saline (e.g., comprising sodium phosphate), a tris buffer saline, an acetate buffer saline (e.g., comprising sodium acetate), a bicarbonate buffer saline, a carbonate buffer saline, a citrate buffer saline (e.g., comprising sodium citrate), and a succinate buffer saline (e.g., comprising sodium succinate).

In several embodiments the nano-emulsion composition may have a pH from 4.0 to 9.0, in particular from 7.2 to 7.6. A pH within these ranges may also contribute to the organization and stabilization of the NEs.

A saline or a buffered aqueous solution and in particular a saline-containing buffered solution may be preferred as such solution also may mimic physiological conditions and may be more adequate as pharmaceutical formulations.

The present disclosure also relates to a lyophilized composition, wherein the composition is an oil-in-water nano-emulsion composition as described herein. Such lyophilized compositions comprise the solid matter, also referred to as solid residue, of the nano-emulsion after removal of water, e.g. by lyophilization. Advantageously, it has been found that such lyophilized compositions retain the structure of the oily phase and can be reconstructed into a nano-emulsion as described herein by addition of water, e.g. de-ionized water, or an aqueous solution, e.g., a buffered aqueous solution such as those described above for the nano-emulsion. A lyophilized composition has advantages for storage and transport, and water or an aqueous phase can simply be added to the lyophilized composition before use.

The present disclosure also relates to a formulation, and particularly, a pharmaceutical formulation comprising a nano-emulsion composition or a lyophilized composition as described herein.

As used herein, "pharmaceutical formulation" means a composition or active ingredient, mixed with at least one pharmaceutically acceptable ingredient, such as, but not limited to, e.g., water or an aqueous solution as defined above for the aqueous medium.

The nano-emulsion composition or the lyophilized composition mixed with an aqueous medium may be as such a pharmaceutical formulation or may comprise additional pharmaceutically acceptable ingredients (also referred to as pharmaceutical excipients) other than water or an aqueous solution as defined above. Such additional excipients may be dissolved, suspended or mixed in said water or aqueous solution of the aqueous medium, or may be mixed into a lyophilized composition as described herein, before or after addition of the aqueous medium. A pharmaceutical excipient may confer to the formulation improved stability, improved absorption, improved solubility and/or enhancement of the activity of the active ingredient, such as a therapeutic enhancement of a therapeutic agent. A pharmaceutical excipient may be selected from vehicles (e.g., petrolatum, dimethyl sulfoxide or mineral oil), carriers, stabilizers, diluents, dispersing agents, suspending agents, thickening agents, lyo-protectants (e.g., mannitol, lactose, polyethylene glycol or polyvinylpyrrolidone), collapse temperature modifiers (e.g., dextran, Ficoll^{™} or gelatin) and the like.

**The** present disclosure also relates to a method for the preparation of the nano-emulsion composition, a lyophilized composition or a formulation comprising the same as described herein. Methods of preparation as described below can include the steps described herein, and these maybe be, but not necessarily, carried out in the sequence as described. Other sequences, however, also are conceivable. Moreover, individual or multiple steps may be carried out either in parallel and/or overlapping in time and/or individually or in multiply repeated steps. Furthermore, the methods may include additional, unspecified steps.

For example, nano-emulsion compositions may be made by the solvent-displacement technique. Briefly, a water-miscible organic solution such as ethanol containing hydrophobic components - such as lipids (e.g., the ionizable cationic lipid (a), the permanently cationic lipid (b), if present the additional lipid (j)), oils (e.g., the oil (c)), surfactants (e.g., the surfactant (d) such as the non-ionic surfactants) and, if present, the PEG-lipid (g) - can be mixed with an aqueous solution, containing hydrophilic components, usually the active ingredient (e). **The** mixing process can be fast, in a dropwise manner, or at a controlled speed, usually through moderate stirring or microfluidic devices. During the formulation process, hydrophobic components precipitate in a controlled manner. Typically, the nano-emulsions may comprise the droplets in an aqueous phase or aqueous medium, e.g., the droplets being dispersed in the aqueous medium (e.g., in water or the aqueous solution as described above) forming an O/W emulsion.

Nano-emulsions may be subsequently subjected to a step to provide the droplets with a coating. For instance, a polymeric solution (e.g., comprising an amphiphilic polymer (h) and/or a hydrophilic polymer (i) as defined above) may be mixed with the previously formed NEs, allowing the formation of electrostatic interactions between the positive or negative surface of the droplets of the nano-emulsion to the complementary-charged polymer or rely on hydrophobic interactions. See, e.g., Calvo et al. (1997) Colloid Polym. Sci. 275:46-53; see also, Wang et al. (2016) Nanomaterials 6:1-18 and Crecente-Campo et al. (2019) Bioeng. Transl. Med. 4:38-50.

Accordingly, a method for preparing a nano-emulsion composition as described herein may comprise, e.g., the steps of:
1) dissolving (a) an ionizable cationic lipid, (b) a permanently cationic lipid, (c) an oil, (d) a surfactant and, if present, (g) a PEG-lipid and (j) an additional lipid selected from a phospholipid, a fatty acid, a fatty acid derivative; and a fatty amine, in a water-miscible organic solvent, preferably in ethanol, to provide a water-miscible organic phase;
2) dissolving (e) an active ingredient in an aqueous medium (f) (e.g., water or an aqueous solution, preferably a buffered aqueous solution), to provide an aqueous phase; and
3) mixing the water-miscible organic phase and the aqueous phase to provide the nano-emulsion.

The presence of the active ingredient in the aqueous solution of step 2) may result in entrapping the active ingredient within the droplets of the nano-emulsion obtained in the mixing step (3).

Additionally, or alternatively, step (3) may include adding an active ingredient to the nano-emulsion and mixing. Including an active ingredient in step (3) may provide, e.g., droplets in the nano-emulsion with an active ingredient on their surface (in addition to or alternatively to the active ingredient entrapped in the droplets of the nano-emulsion obtained if the active ingredient is present in the aqueous phase obtained in step (2)).

The method may comprise a subsequent step (3') comprising coating the droplets in the nano-emulsion obtained in step (3). Coating of step (3') may comprise mixing the nano-emulsion with a polymeric solution. The polymeric solution may comprise a polymer as described above, e.g., an amphiphilic polymer (h) and/or hydrophilic polymer (i), dissolved in a suitable solvent, such as water or an aqueous solution, preferably water or a buffered aqueous solution.

Mixing of the water-miscible organic phase and the aqueous phase of step (3) generally provides the nano-emulsion.

The droplets in the aqueous phase of the nano-emulsion obtained comprise the ionizable cationic lipid (a), the permanently cationic lipid (b), the oil (c) and the surfactant (d), the active ingredient (e) and, if present, any PEG-lipid (g), amphiphilic polymer (h) and/or the hydrophilic polymer (i) and the additional lipid (j). The distribution of the different components within the droplets or in the surface of the droplets may be as described above. The aqueous medium constituting the aqueous phase may be water or an aqueous solution a saline, a buffered aqueous solution, or a buffered saline solution, e.g., as described above for the formulation.

When the method is for the preparation of a lyophilized composition as described above, the method may further comprise:
4) lyophilizing the nano-emulsion provided in step 3).

Lyophilizing may be performed by means known in the art.

If the method comprises a subsequent coating step (3') lyophilizing may be performed after said coating step (3')

The lyophilized composition may be reconstructed into a nano-emulsion by addition of an aqueous medium, e.g., water or a buffered solution such as those described above for the nano-emulsion composition. A method for the preparation of a reconstructed nano-emulsion may therefore comprise, adding an aqueous medium to a lyophilized composition as described herein. The method for preparing a reconstructed nano-emulsion may also comprise A) the preparation of the nano-emulsion composition as described above, comprising steps 1-3 (and optionally step 3'), B) the preparation of the lyophilized composition as described above, comprising step 4, and C) the preparation of the reconstructed nano-emulsion comprising 5) adding an aqueous medium to the lyophilized composition obtained in step 4).

The nano-emulsion composition or the lyophilized composition may be further processed to add further ingredients such as additional active ingredients or pharmaceutical excipients, or to adapt it to a form suitable for a specific route of administration for the treatment of a disease or disorder, as described above.

What is described above for the nano-emulsion and the formulation also applies to the methods of their preparation as described herein.

The present disclosure also relates to a nano-emulsion composition, a lyophilized composition or a formulation as described herein for use in the treatment (including the preventive treatment) of a disease or a disorder. Particularly suited may be diseases or disorders susceptible to be treated with active ingredients that can be encapsulated in NEs as described herein, such as nucleotides including RNA, e.g., for RNA-therapeutics.

Preferably a nano-emulsion composition (including a reconstructed nano-emulsion from a lyophilized composition) or a formulation as described herein may be for the treatment or prevention of CNS-associated diseases or disorders, PNS-associated diseases or disorders, autoimmune diseases or disorders, liver-associated diseases or disorders, lung-associated diseases or disorders, spleen-associated diseases or disorders, immunological diseases or disorders, cancers, and infectious diseases or disorders.

Said treatment or prevention of a disease or disorder may include cell therapy, whereby a nano-emulsion or formulation as described is used to modify cells, e.g., *in vitro,* or *in vivo,* for cell therapy, e.g., for CAR-T cell therapy. Such cells modified by using a nano-emulsion or formulation as described may then be for use of the treatment or the prevention of a disease or a disorder, e.g., by administration to a subject suffering from the disease or disorder, to a healthy subject or to a subject, e.g., susceptible of contracting a disease or disorder.

In some embodiments, the prevention of a disease or disorder may be achieved by a nano-emulsion composition or formulation as described herein for use as a vaccine, e.g., for the prevention of cancer or infectious diseases or disorders.

The present disclosure also relates to a method of treatment of a disease or a disorder, the method comprising administering a nano-emulsion composition, a lyophilized composition or a formulation as described herein to a subject suffering from the disease or disorder.

The present disclosure also relates to methods of preventive treatment comprising to administering said composition or formulation to a healthy subject or to a subject, e.g., susceptible of contracting a disease or disorder.

In some embodiments, the disease or disorder is a CNS-associated or PNS-associated disease or disorder, and the subject to be treated or preventively treated may be an individual having a CNS- or PNS-associated disease or disorder, a healthy individual or an individual susceptible of contracting such a disease or disorder. For such treatments the delivery of the active ingredient across the BBB and/or throughout the CNS may be needed and can be achieved with nano-emulsions as described herein.

In other embodiments the disease or disorder is a liver-associated disease or disorder, a lung-associated disease or disorder or a tumor-related disease or disorder. In these cases, a nano-emulsion composition or formulation thereof may be either administered systemically (e.g., intravenously) or locally (e.g., intratumorally).

The composition or formulation for use in the treatment of a disease or a disorder may be for use in a treatment comprising administering the composition or formulation to a subject as described for the methods of treatment.

Examples of CNS-associated diseases and disorders include, but are not limited to, neurodegenerative diseases, disorders and/or conditions including, but not limited to, Alzheimer's disease, Parkinson's disease, Huntington's disease, amyotrophic lateral sclerosis, and spinocerebellar ataxias, and cancers such as glioblastoma.

Examples of liver-associated diseases or disorders include, e.g., liver fibrosis, liver-specific cancers.

Examples of lung-associated diseases or disorders include, e.g., lung-specific cancers.

Examples of diseases that allow for immunological therapy, including cell therapies include, e.g., hematological and solid tumors.

A nano-emulsion composition or a formulation as described herein can be administered to a subject by any route, including enteral (e.g., oral) or parenteral routes. For instance, may be administered intravenous (IV), subcutaneous (SC), intracerebroventricular (ICV), intracisternal magna (ICM), intraparenchymal (IPM), intrathecal (ITH), intrastriatal (IST), intramuscular, intra-arterial, intramedullary, transdermal, interdermal, rectal, intravaginal, intraperitoneal, topical (as by powders, ointments, creams and/or drops), mucosal, nasal, e.g., nose-to-brain (N-to-B), buccal, sublingual; by intratracheal instillation, bronchial instillation and/or inhalation; and/or as an oral spray, nasal spray, and/or aerosol. Specifically contemplated routes are oral administration, IV injection (e.g., systemic intravenous injection), SC injection, ICV injection, ICM injection, IPM injection, ITH injection, IST injection, N-to-B, or a combination thereof. In some instances, the administering can be via an ICM injection, an intracerebroventricular ICV injection, intrathecal injection or N-to-B delivery.

Generally, the most appropriate route of administration will depend upon a variety of factors including, but not limited to, the nature of the agent (e.g., its stability in the environment of its administration and/or intended target) and/or the condition of the individual (e.g., whether the subject is able to tolerate oral administration).

In some instances, the nano-emulsion composition or the formulation as described herein are suitable for administration to, for example, the CNS of an individual via ICV injection, ICM injection, ITH injection, IST injection or N-to-B delivery.

The treatment of the disease or the disorder may comprise administering the subject an effective amount of a nano-emulsion composition or a formulation including the same as described herein. An effective amount may depend on the type of active ingredient, route of administration and the characteristics of the subject. The active ingredient-particle complexes herein are generally effective over a wide dosage range. Exemplary doses can be milligram (mg), microgram (µg), nanogram (ng) or picrogram (pg) amounts per kilogram (kg) of the subject. In this manner, a dose can be from about 1 µg to about 200 mg per kg of the subject.

In some instances, the method also can include a step of administering to the individual at least one additional active ingredient, e.g., an effective amount of said additional active ingredient.

The at least one additional active ingredient is an active ingredient that is distinct from the active ingredient (d) of the nano-emulsion composition as described herein. For instance, the additional active ingredient can be an antibody (or fragment thereof), a peptide, or a therapeutic oligonucleotide (i.e., one that is distinct from, e.g., a therapeutic nucleotide (d) of the nano-emulsion composition). The additional active ingredient may be, e.g., an adjuvant to enhance the activity of the active ingredient, for instance the active ingredient may be a nucleotide such as RNA and the additional active ingredient may be an adjuvant, which may also be RNA. The additional active ingredient may also be a small molecular weight drug in case of a combination therapy.

The at least one additional active ingredient can be administered simultaneously, separately or sequentially with the nano-emulsion composition or the formulation including the same as described herein. In some instances, the at least one additional active ingredient can be administered with a frequency same as the nano-emulsion composition or formulation including the same as described herein or with a frequency distinct therefrom. Likewise, the at least one additional active ingredient can be administered via a route same as the nano-emulsion composition or the formulation including the same as described herein or via a route distinct therefrom.

The present disclosure further relates to the use of a nano-emulsion composition or a formulation comprising the same as described herein for use in the manufacture of a medicament, in particular a medicament of treatment of CNS-related diseases or disorders, PNS-related diseases or disorders, autoimmune diseases or disorders, liver-associated diseases or disorders, lung-associated diseases or disorders immunological diseases or disorders, cancers and infectious diseases or disorders.

Advantages of nano-emulsion compositions as described herein include, e.g.:
- to have long-term stability at low temperatures (e.g., at least about 1 month at about 4°C);
- to have a low toxicity profile and have a dose-dependent transfection efficiency across different model cell lines;
- to display *in vivo* efficacy and biocompatibility in diverse target areas, including upon direct brain delivery (i.e., can encapsulate, protect and deliver a therapeutic nucleotides to the CNS), where they exhibit transfection specificity for neurons and/or microglia cells; and
- to display prominent distribution throughout the brain, indicated by the transfected area/volume including distribution throughout the brain parenchyma; and
- to display *in vivo* efficacy and biocompatibility upon intravenous administration (e.g., can encapsulate, protect and deliver therapeutic oligonucleotides and polynucleotides to the liver and extrahepatic tissues).

Unless defined otherwise, all technical and scientific terms used herein have the same meaning as commonly understood by one of skill in the art to which the disclosure pertains. Although any methods and materials similar to or equivalent to those described herein can be used in the practice or testing of the methods herein, the preferred methods and materials are described herein.

Additionally, reference to an element by the indefinite article "a" or "an" does not exclude the possibility that more than one element is present, unless the context clearly requires that there be one and only one element. The indefinite article "a" or "an" thus usually means "at least one."

In this text, the term "includes", "comprises" and derivations thereof (such as "including", "comprising", etc.) should not be understood in an excluding sense, that is, these terms should not be interpreted as excluding the possibility that what is described and defined may include further elements, steps, etc. On the other hand, these terms, even if they are not limiting to the specifically recited items they also encompass embodiments limited to the specifically recited items, which may in turn be designated by the expression "consisting of". The use of "selected from" refers to one or more of the recited items.

On the other hand, the disclosure is obviously not limited to the specific embodiment(s) described herein but also encompasses any variations that may be considered by any person skilled in the art (for example, as regards the choice of materials, dimensions, components, configuration, etc.), within the general scope of the invention as defined in the claims.

The present disclosure is further illustrated by the following examples without being limited thereto or thereby.

### EXAMPLES

### Example 1: preparation of NEs

Nano-emulsion compositions (NEs) were prepared by using a solvent-displacement technique.

The compositions of the NEs included
- 8 - 60 mol% of an ionizable cationic lipid (C12-200 from Corden Pharma in for NE1, 306Oi10 from Echelon Biosciences for NE2, and SS-OP from NOF Europe for NE3),
- 8 - 40 mol % of a permanently cationic lipid (DOTAP chloride salt from Merk/Avanti Polar Lipids in all NEs),
- 0 - 70 mol % of a phospholipid (DOPE from Merk/Avanti Polar Lipids in all NEs),
- 20 - 80 mol% of an oil (Vit E from BASF in all NEs) and
- 0.1 - 40 mol% of a surfactant (T80 from Merck Millipore in all NEs), and
- 0.01 - 15 mol% and DSPE-PEG (from Lipoid in all NEs).

The NEs were prepared as such or were prepared to encapsulate RNA.

In some experiments the encapsulated RNA was a commercially available mRNA encoding green fluoresecent protein (GFP), also referred to as mRNA GFP or mGFP, and was used to visualize the delivery of the mRNA *in vitro* and *in vivo,* as detailed below. The mRNA GFP used was PureBoost^{™} EGFP mRNA from Cellerna. In other experiments, the encapsulated RNA was an mRNA encoding firefly luciferase (Luc), PureBoost^{™} FLuc mRNA from Cellerna also referred to as mRNA Luc or mLuc, and was used to visualize the delivery of mRNA *in vivo,* as detailed below.

In other experiments, the NEs were prepared to encapsulate other target RNAs such as therapeutic RNAs including small interfering RNAs (siRNAs), which can regulate the expression of a target mRNA, by, e.g., silencing it. The delivery of siRNA was assessed in *vitro,* as detailed below. The model siRNA was a commercially available siRNA silencing GFP, referred to as siRNA GFP or siGFP (from Biospring), and was used to quantify the delivery efficiency of siRNA *in vitro.* Other, therapeutic siRNAs were used in disease-relevant models, evaluating the silencing of the expression of target proteins.

### Preparation and characterization of the NEs

A water-miscible organic solution of hydrophobic components including the lipids and the oils and hydrophobic surfactants in ethanol was mixed with an aqueous solution of the hydrophilic components including the mRNA such as mGFP or a therapeutic RNA such as a small interfering RNA (siRNA).

Mixing can be performed in a fast manner, by controlling the stirring speed or in bulk mixing or by microfluidic mixing. During the formulation process, the water-miscible organic solution of hydrophobic components was rapidly mixed with and diluted by an aqueous solution of hydrophilic components, including the RNA, whereby the components precipitated in a controlled manner. Specifically, in case of NEs 1-3, the aqueous and organic solutions were mixed in a 4:1 volume ratio of aqueous solution to organic solution, ratios between 1:1 and 6:1 were explored. In case of bulk mixing, the aqueous phase was stirred at a speed between 500 and 1500 RPM while the organic phase was rapidly pipetted onto the stirring liquid. In case of microfluidic mixing flow speeds of 1 - 15 mL/minute were explored, most optimally being 9 mL/minute. This was consistent across several microfluidic devices evaluated, including a NanoAssemblr bench-top micromixer (Precision NanoSystems Inc.), Helix Biotech Mixer (Helix Biotech) and TAMARA (Inside Tx). In all specific cases, total material:RNA weight ratio was fixed at 19:1, resulting in a 5% w/w RNA loading, the total material including all components (a), (b), (c), (d), (e), (g) and (j) as detailed in Table 1 below.

Post-formulation, the nano-emulsions are transferred to a higher ion strength with phosphate buffer saline (PBS) or tris buffer saline (TBS) as a neutral buffer.

The final suspension obtained had a concentration of mRNA (e.g., mGFP), siRNA (e.g., siGFP) or therapeutic RNA in a range of 0.025 to 2.0 mg/mL with respect to the volume of the suspension.

The NEs were characterized by determining the average particle size of the droplets of the NE, the polydispersity index (PDI), their zeta potential (ZP) or ζ potential and their encapsulation efficiency (EE).

The average particle size, in terms of the hydrodynamic diameter of the droplets, and the PDI was determined by dynamic light scattering with a Zetasizer^{®} Nano ZS (Malvern Instruments).

The ZP was measured in terms of mean electrophoretic mobility values, measured by laser Doppler electrophoresis with the same equipment. Particle size and PDI measurements were performed after diluting the samples 5x to 200x in salt-containing neutral buffer, depending on the concentration of the final suspension. ZP characterization was obtained after dilution of samples 5x to 200x in salt-containing neutral buffer, depending on the concentration of the final suspension

To quantitatively determine EE% and the concentration of the RNA agent encapsulated by the NEs, a Quant-iT RiboGreen RNA assay kit (Invitrogen) was used. Briefly, samples of the NEs comprising the encapsulated the RNA, were diluted 50-fold in TE buffer (1x), followed by a 2-fold dilution with Triton X (Triton-X-100, Sigma-Aldrich and incubation at 37°C for 30 min), which induced the disruption of the NEs. Subsequently, samples were diluted 10-fold with TE buffer (1x). A standard curve was prepared using the RNA agent which was encapsulated at concentrations between 0.1 µg/mL-1 µg/mL in the presence of Triton X. Following manufacturer instruction, RiboGreen reagent was diluted 200-fold in TE buffer (1x) and was added to an equal volume of sample, leading to a final volume of 200 µL. Samples and the standard curve were transferred to a Corning^{®} 96-well black polystyrene microplate. Fluorescence intensity was assessed using a Synergy H1 Microplate Reader (BioTek Instruments) using excitation at 485 nm and emission at 530 nm. The standard curve (r² ≥ 0.99) was used to determine the RNA agent concentration of the sample. The EE% of the RNA was calculated according to the following equation: $EE% = \frac{{\left[RNA\right]}_{disrupted}}{{\left[RNA\right]}_{theoretical}}$ (Equation 2) where [*RNA*]*_{disrupted}* is the RNA agent concentration determined after treating the NE with the disrupting agent, and where [*RNA*]*_{theoretical}* is the theoretical total RNA agent concentration of the NE.

The amount of RNA agent in each sample was calculated using a standard curve generated with known concentrations (4 µg/mL to 320 µg/mL - r2 ≥ 0.99). The EE% of RNA agent was calculated according to Equation 2. Each analysis was done at least in duplicate.

The apparent pKa of the formulation was measured via the TNS (6-(p-Toluidino)-2-naphthalenesulfonic acid) assay. NEs were diluted in 0.9% (w/v) NaCl to a final concentration of 1 µg/mL and incubated for 5 min in a series of buffers containing 20 mM citrate, MES, HEPES, and boric acid in 0.9% NaCl, with pH values ranging from pH 3.0 to 11.0 and a final TNS concentration of 16µM. The mean fluorescence intensity was measured in a black-bottom 96-well plate (Synergy H1 microplate reader, BioTek, VT, USA) at λEx = 321 nm and λEm = 447 nm at 37°C. The apparent pKa was defined by the pH at half-maximum fluorescence.

To assess stability, colloidal stability of the NEs encapsulating the RNA agents samples was assessed upon storage at 4°C for up to 1 month. Besides visual inspection of the samples, to identify macroscopic changes in their appearance, color or the presence of aggregates, measurements of particle size, PDI, Z-potential and RNA agent association efficiency were performed. Additionally, the stability in biologically relevant media, or media mimicking those, was assessed. Nano-emulsion stability was confirmed in FBS (10-50%) containing salt-containing buffer, as well as in human plasma. Functional stability was assessed via *in vitro* evaluation upon storage at 4°C for 4 weeks. The sample in solution was considered statable if the change in the average diameter is inferior to 10%, as compared to the initial sample.

### Composition and properties of the NEs

NEs were prepared with a ratio of its components as indicated in the table below
- of 10.55: 10.55: 19.6: 51.3: 7: 1 (ionizable cationic lipid: permanently cationic lipid: phospholipid: oil: surfactant: PEG-lipid), also referred to as NE-C12 owing to the ionizable cationic lipid used.
- of 21.42: 12.93: 18.66: 40.0: 6: 1 (ionizable cationic lipid: permanently cationic lipid: phospholipid: oil: surfactant: PEG-lipid), as indicated in the table above, also referred to as NE-306 owing to the ionizable cationic lipid used.
- of 32.52: 12.73: 8.75: 36.0: 9: 1 (ionizable cationic lipid: permanently cationic lipid: phospholipid: oil: surfactant: PEG-lipid), as indicated in the table above, also referred to as NE-SS9 owing to the ionizable cationic lipid used.
- of 20.38: 18.79: 0.0: 50.03: 8.9: 1 (ionizable cationic lipid: permanently cationic lipid: phospholipid: oil: surfactant: PEG-lipid), as indicated in the table, also referred to as NE-306-ppl-free, owing to the type of ionizable cationic lipid and the absence of phospholipid.

**Table 1: NE compositions**

| | **NE1 (NE-C12)** | **NE2 (NE-306)** | **NE3 (NE-SS9)** | **NE4 (NE-306-ppl-free)** |
|---|---|---|---|---|
| a) ionizable cationic lipid (mol %) | C12-200 (10.55) | 306Oi10 (21.42) | SS-OP (32.52) | 306Oi10 (20.38) |
| b) permanently cationic lipid (mol %) | DOTAP (10.55) | DOTAP (12.93) | DOTAP (12.73) | DOTAP (18.79) |
| j) phospholipid (mol %) | DOPE (19.6) | DOPE (18.66) | DOPE (8.75) | DOPE (0) |
| c) oil (mol %) | Vit E (51.3) | Vit E (40.0) | Vit E (36.0) | Vit E (50.93) |
| d) Surfactant | T80 (7) | T80 (6) | T80 (9) | T80 (8.9) |
| e) Encapsulated RNA agent | mGFP or siRNA | mGFP or siRNA | mGFP or siRNA | mGFP or siRNA |
| g) PEG-lipid (mol %) | DSPE-PEG (1) | DSPE-PEG (1) | DSPE-PEG (1) | DSPE-PEG (1) |
| size - average diameter (nm) | 58 ± 3 | 60 ± 2 | 56±4 | 65 ± 2 |
| polydispersity index (PDI) | 0.20 | 0.18 | 0.18 | 0.14 |
| zeta potential ZP (mV) | - 0.8 ± 0.2 | - 1.3 ± 0.6 | - 0.5 ± 0.1 | - 0.4 ± 0.2 |
| EE | 100 % | 100 % | 100 % | 100 % |
| Stability | Stable | Stable | Stable | Stable |

The general structure of NEs obtained can be observed obtaining cryoTEM images as shown in Figure 1 for NE containing mRNA (Figure 1A) or siRNA (Figure 1B)

### Example 2: In vitro performance of NEs in the delivery of mRNA encoding GFP

SH-S5Y5 neurons (a cloned subline of a neuroblastoma cell line from a metastatic bone tumor) were treated *in vitro* with a suspension NEs 1, 2 and 3 encapsulating mRNA of GFP as a model of active ingredient, having an initial concentration of mGFP of 0.1 mg/mL with respect to the volume of the suspension, and of 5 wt.% with respect to the total mass of (a), (b), (c), (d), (e), (g) and (j) (in table 1). The appropriate amount of NEs comprising mGFP was applied to the cells to achieve different doses: 5 µg, 2.5 µg, 1.25 µg, 0.625 µg, 0.3125 µg, of a total load of mGFP administered to the cells. A total of 150,000 cells were seeded per well in a flat bottom 12-well plate and allowed to adhere for 24 h.

Cells were treated with NE-mGFP in Opti-MEM^{™} (Gibco^{™}, Thermo Fisher, MA, USA) at the above amount of mGFP per well, for 4 h. Nanocarriers were then removed and replaced with a complete medium. Cells were incubated for another 20 h. For transfection efficiency assessment, cells were trypsinized, harvested, and fixed with 1% (*w*/*v*) formaldehyde in PBS. The percentage of GFP-positive cells and mean fluorescence intensity were analyzed by flow cytometry (BD Accuri, BD Biosciences, NJ, USA).

The *in vitro* toxicity of the NEs 1-3 was tested on the viability of treated neurons after treatment. Cell viability was measured by absorbance-based MTT assay (3-(4, 5-dimethyl-2-thiazolyl)-2, 5-diphenyl-2H-tetrazolium bromide; Sigma-Aldrich, MO, USA). Briefly, cells were incubated with MTT reagent (1 mg/mL) for 4 h at 37 °C. After removing the MTT reagent, the resulting formazan crystals were dissolved in acidic isopropanol and quantified in a plate reader at 544/590 nm (Infinite 200 PRO, Tecan, Männedorf, Switzerland). The tests were performed alongside untreated cells.

The results are shown for untreated cells and for cells 24h post-treatment (N=3) for the following NEs
- NE 1 (NE-C12) in Figures 2A, 2B and 2C;
- NE 2 (NE-306) in Figures 3A, 3B, 3C and 3D;
- NE 4 (NE-309-ppl-free) in Figure 3D; and
- NE 3 (NE-SS9) in Figures 4A, 4B and 4C.

Figures 2-4 A being the *in vitro* toxicity evaluation expressed in terms of % viability, Figures 2-4 B and 2-4 C showing the transfection efficiency expressed as the percentage of GFP positive (GFP+) cells (%) in B, and expressed as the mean fluorescence intensity (MFI) of GFP in C.

The effects obtained were compared to those obtained for untreated cells and for cells treated with TurboFect^{™} transfection agent (from Thermo Scientific^{™}) for the delivery of the mRNA of GFP, used in a dose 5 µg of mRNA GFP per the same number of cells, with preparation of the reagent according to the manufacturer instructions. Briefly, the respective volumes of TurboFect^{™} transfection reagent and mRNA GFP at the desired concentration, both prediluted in culture medium, were mixed and incubated before added to the cells.

As it can be seen the results of Figures 2-4, the NEs 1-3 show no toxicity, with a viability of the treated cells which is equivalent to the non-treated cells (for showing a non-significant (ns) difference on the % viability), Figures 2A, 3A and 4A. On the other hand, the NEs 1-3 were found to exhibit potent transfection of SH-S5Y5 neurons, for both percentage of GFP positive cells as well as for GFP MFI (Figures 2B, 3B and 4B and Figures 2C, 3C and 4C).

Transfection efficiency was also tested for NE-306 and NE-306-ppl-free loaded with siRNA GFP (siGFP), for HeLa GFP cells, by measuring the percentage of GFP+ cells as described above. In this experiment, the more efficient the transfection of the silencing RNA for GFP the lower the percentage of GFP+ cells. The results are shown in Figure 3D for phospholipid free NE-306-ppl-free (left) and phospholipid containing NE-306 (right). As can be observed the presence of phospholipid resulted in a lower percentage of GFP+ cells, therefore demonstrating a higher transfection efficiency when the phospholipid was present.

A data set generated in a Design of Experiment (DoE) study indicated that the presence of a phospholipid was significantly contributing to both, enhanced transfection efficiency and nano-emulsion stability in biologically relevant media. Specifically, the design space explored 0-20 mol% of the phospholipid DOPE, confirming that increased phospholipid content enhanced both these parameters (data not shown). Figure 3D is representative of the improvement observed in the presence of the phospholipid.

### Example 3: In vivo performance of NEs in the delivery of mRNA encoding GFP

### Injection into the stratium

### Example 3A:

*In vivo* experiments were conducted as follows:

| | |
|---|---|
| • Animal: | Sprague Dawley (SD) rats |
| • Administration: | intrastriatal, in both hemispheres |
| • Volume injected: | 3 µL of a suspension of NEs 1-3 |
| • mRNA concentration: | 1.0 mg/mL of mGFP per volume of suspension, injecting a total of 3 µg mGFP |
| • Time point of analysis: | 24h post-injection |
| • Control: | injection of same volume of PBS (free of NEs) |

Considering that the *in vivo* experiments were performed for three different NEs (NEs 1-3) in parallel (including injection of different NEs types into right and left hemispheres of the same animal), the data is presented together.

Firstly, Figure 5 shows the *in vivo* mGFP transfection efficiency evaluation of the NEs 1-3 (NE-C12, NE-306 and NE-SS9), expressed as mean fluorescence intensity (MFI) of GFP upon injection of 3 µg mRNA GFP into the striatum of SD rats. Measured 24h post-treatment. N>3.

Secondly, the *in vivo* colocalization GFP signal induced by the NEs 1-3 was studied with specific markers for different types of cells: neurons (including anti-GFP, anti-β-Tubulin and Hoechst markers), microglia (including GFP, anti-IBA1 and Hoechst markers), and astrocytes (including GFP, anti-GFAP and Hoechst markers), upon injection of 3 µg mRNA GFP into the striatum of SD rats. Briefly, administration of NEs was performed in male Sprage-Dawley rats (3 animals in the NE-mGFP group and 1 animal in the PBS, control group) deeply anesthetized with ketamine (50 mg/kg) and medetomidine (0.4 mg/kg). Anesthetized animals were mounted in a stereotaxic frame (Kopf Instruments, CA, USA) and were injected with 3 µg of mGFP encapsulated onto NEs, in a total volume of 3 µL. The solution was injected using a 5- µL Hamilton syringe, coupled to a motorized injection (Stoelting), at a rate of 0.5 µL/min. Stereotaxic coordinates were A/P: 0.8 mm, M/L: 3.0 mm and D/V: 5.0 mm. The needle used for the administration was left in place for an additional 5 min before withdrawal to avoid NEs reflux. After 24 h, animals received an anesthetic overdose and they were sacrificed by decapitation; their brains were rapidly removed, cryoprotected, and cut into coronal tissue sections using a sliding microtome. Tissue sections were processed for immunofluorescence labeling as follows. Free-floating tissue sections were pre-incubated in KPBS-1% BSA with 5% normal donkey serum (Sigma-Aldrich, MO, USA) and 0.03% Triton X-100 for 60 min at room temperature. Initially, single immunofluorescence was performed to identify cells capable of expressing GFP after mGFP transfection. The different brain cell types were labeled with primary antibodies against class III β-tubulin (β III-tubulin, 1:750, T8660, Sigma-Aldrich, MO, USA) as a neuronal maker, or glial fibrillary acidic protein (GFAP, 1:500, MAB360, Merk Millipore, MA, USA) as an astrocytic marker, or ionized calcium-binding adaptor molecule 1 (Iba1, 1:500, 019-19,741, Wako Chemicals, Neuss, Germany) as a microglial maker. Cell nuclei were marked with the DNA-binding dye Hoechst 33342 (1:2000, 62,249, Sigma-Aldrich, MO, USA). Immunoreaction was visualized with the fluorescence secondary antibody Alexa Fluor 568- conjugated donkey anti-rabbit IgG (1:200, Molecular Probes, OR, USA) or Alexa Fluor 488-conjugated donkey anti-mouse IgG (1:200, Molecular Probes, OR, USA). Sections were mounted on gelatin-coated slides and cover-slipped with Immumount (Thermo-Shandon). Colocalization of these markers was assessed by confocal laser microscopy (AOBS-SP5X; Leica Microsystems Heidelberg GmbH, Mannheim, Germany). Then, considering the auto-green fluorescence depicted by neurons, the decision was made to perform a double immunofluorescence against GFP (anti-GFP, 1:1000, C10362, Molecular Probes, OR, USA) and β III-tubulin, followed by the incubation with fluorescence secondary antibodies Alexa Fluor 568-conjugated donkey anti-rabbit IgG (1:200, Molecular Probes, OR, USA) and Alexa Fluor 488-conjugated donkey anti-mouse IgG (1:200, Molecular Probes, OR, USA), respectively. Then, the preparation of the section was performed as previously described. The results are shown in Figure 6A for NE1 (NE-C12), Figure 6B for NE2 (NE-306) and Figure 6C for NE3 (NE-SS9), including images for each marker and the corresponding merge image. The results indicate co-localization for all cell types and, therefore, they confirm the *in vivo* specificity of the NEs for neuronal cells.

The localization of GFP in the brain upon intrastriatal administration of the NEs encapsulating mGFP was performed by a brain mapping of GFP on the coronal plane. Briefly, following the immunoreaction that visualized the fluorescence, as indicated above, confocal laser microscopy was utilized for brain mapping. Hundreds of images were taken at high magnification and subsequently stitched together to obtain the brain map.

The results are shown in Figure 7 for specimens upon intrastriatal injection of 3 µL of a suspension of NEs 1-3 at a concentration 1.0 mg/mL mGFP (corresponding to a total dose of 3 µg mGFP) by visualizing the GFP and is compared to specimens upon injection of with PBS (without NEs). The images show the transfected area which glows upon intrastriatal injection of NEs but shows no glow upon injection of PBS, showing a higher intensity for the NE-SS9 NE3 when compared to the NE-306 and NE-C12 NEs 1 and 2.

In Figure 8A, lack of fluorescence due to lacking GFP expression upon administration of PBS is observed and functions as control. In Figure 8B and 8C the fluorescence generated by the expression of GFP upon administration of NEs encapsulating GFP-mRNA is observed: for NE-SS9 (left) and NE-306 (right) in Figure 8B, and for NE-SS9 (left) and NE-C12 (right) in Figure 8C.

### Injection into mice tail

### Example 3B:

*In vivo* experiments were conducted as follows:

| | |
|---|---|
| • Animal: | C57BL/6 mice |
| • Administration: | intravenous, tail vein |
| • Volume injected: | 100 µL of a suspension of NEs of examples 2-3 |
| • mRNA concentration: | 0.1 mg/mL of mGFP per volume of suspension, injecting a total of 10 µg mGFP |
| • Time point of analysis: | 24h post-injection |
| • Control: | injection of same volume of PBS (free of NEs) |

Mice were injected with 100 µL of a suspension of NE2 (NE-306) and of NE3 (NE-SS9) into a vein of their tail. Briefly, eight-week-old C57BL/6 mice received a single injection via the tail vein with vehicle (PBS), example formulation 2 and 3, injecting a total of 10 µg mGFP per animal. The animals were sacrificed 24 hours after injection. Nuclei of liver tissue samples were stained with DAPI and representative immunofluorescence for GFP obtained after delivery of mGFP with NEs 2 and 3 was observed in the cells as intense green fluorescence..

The results are shown in Figures 9A (for NE-306, NE2) and 9B (for NE-SS9, NE3) showing a good distribution of the GFP fluorescence indicating a good delivery of the mGFP in liver cells. White arrows show areas with greatest accumulation of GFP observed as a brighter green areas.

### Example 3C:

*In vivo* experiments were conducted as follows:

| | |
|---|---|
| • Animal: | C57bl6 mice |
| • Administration: | intravenous, tail vein |
| • Volume injected: | 100 µL of a suspension of NE3 |
| • mRNA concentration: | 0.05, 0.1 or 0.2 mg/mL of mLuc per volume of suspension, injecting a total of 5, 10 or 20 µg mLuc |
| • Time point of analysis: | 24h post-injection |
| • Control: | injection of same volume of PBS (free of NEs) |

Mice were injected with 100 µL of a suspension of NE3 (NE-SS9) encapsulating mRNA for luciferase (mLuc) into a vein of their tail.

The resulting Luciferase expression was visualized by bioluminescence via an IVIS system.

The results are shown in Figure 10 (for NE-SS9) showing whole body distribution of Luciferase transfection (Figure 10A for blank with an injection of PBS and an injection of 5 µg of mLuc and Figure 10B for an injection of 10 µg of mLuc and an injection of 20 µg of mLuc) as well as dose-dependency in total flux (Figure 10C), after 6, 24 and 48 hours, for a blank injection of PBS and injection with doses of 5, 10 or 20 µg mLuc. As can be observed the whole bodies injected with PBS (blank) displayed no bioluminiscence (Figure 10A, PBS) whereas the biolumincense was most intense after 6 hours with a maximum intensity of 10⁶ in the central areas of the body and parts of the tail, and above 4x10⁵ in the rest of the body (Figures 10A and 10B). Bioluminiscence decreased with time: after 24h with a maximum intensity of about 4x10⁵ in some areas of the body such as the liver and a lower intensity 2x10⁵ in other areas of the body; and after 48 h with an intensity of 2x10⁵ in the majority of areas of the body with only localized areas with higher intensity of about 4x10⁵. Higher doses showed also higher intensity and especially with time (Figures 10A and 10B).

The change in bioluminescence with doses and with time can also be clearly observed in the graph of Figure 10C for the quantification of the total flux at 6, 24 and 48 hours, for the blank (PBS) and the different doses (5, 10 and 20 µg mLuc).

### Example 4: In vitro performance of NEs in the delivery of a siRNA

NEs comprising a siRNA were prepared as described above for example 1, leading to encapsulated siRNA in NEs including NE-C12 (NE1), NE-306 (NE2) and NE-SS9 (NE3).

The encapsulated RNAs used were siRNA, which are known to down regulate the expression of the corresponding target mRNA. In particular the siRNA used targets and downregulates the expression of a liver-disease driving protein encoding mRNA.

The delivery of the siRNAs was evaluated *in vitro* by measuring the fold change of the expression of the corresponding target mRNA. Briefly, LX-2 hepatic stellate cells were seeded at a density of 50000 cells/well in a 24-well microplate. 24h post-seeding, cells were treated with NEs wherein the aqueous medium was a cell culture medium at indicated concentrations (Figure 11) of siRNA, specifically 25, 50 and 100 nM. 4 hours later, NE containing medium was replaced by regular culture medium. 44 hours later, cells were harvested, and the target mRNA levels were quantified by qPCR.

Figures 11 A-C show the fold change on the expression of the target mRNA upon contacting LX-2 hepatic stellate cells with a suspension of the NEs 1-3 loaded with siRNA, NE-C12 in Figure 11A, NE-306 in Figure 11B and NE-SS9 in Figure 11C. Suspensions having a concentration of the siRNA of 100 nM and 50 nM as well as 25 nM in case of NC-SS9 respectively were used and were compared to cells contacted with the vehicle of the NEs (i.e., PBS in absence of the NEs). The results show a statistically significant reduction in the expression of the target mRNA upon treatment with the NEs.

The delivery of siRNAs was tested in two types of glioblastoma: CNS-relevant cell line A U87 cells and cell line B U373 cells. The cells were contacted with NE 3 (NE-SS9) containing the siRNA, as described above for the LX-2 cells.

Figure 12 shows the results of *in vitro* evaluation of NE-SS9 delivery of the siRNA on target protein levels in CNS-relevant cell line A (left) and cell line B (right). Briefly, U87 and U373 glioblastoma cells were seeded at a density of 50000 cells/well in a 24-well microplate. 24h post-seeding, cells were treated with NEs wherein the aqueous medium was cell culture medium at siRNA concentrations of 40 and 80 nM. 4 hours later, the NE was replaced by regular culture medium. 48, 72, 96 and 120 hours later, cells were harvested and silencing of target protein was quantified by western blotting.

Figure 13 shows the quantification of the above-mentioned kinetic study on duration of target knockdown after single treatment for cell line A (Figure 13A) and cell line B (Figure 13B). Briefly, U87 and U373 glioblastoma cells were seeded at a density of 50000 cells/well in a 24-well microplate. 24h post-seeding, cells were treated with NEs wherein the aqueous medium was cell culture medium at siRNA concentrations of 40 nM. 4 hours later, NE was replaced by regular culture medium. 48, 72, 96 and 120 hours later, cells were harvested and silencing of target protein was quantified by western blotting.

The delivery of siRNA was also assessed in a clonogenic assay upon treatment of glioblastoma cells of cell line B with NE-SS9 (NE3) encapsulating the siRNA, alongside untreated cells and cells encapsulating irrelevant siRNA (which would not affect the expression of the target protein) as controls. Briefly, the cells were treated as indicated above, at siRNA doses of 40 and 80 nM. After 44h, cells were harvested, counted and replated onto a 6-well plate at a density of 200 cells per well. Cells were kept in culture for 10 days, after which the cells were fixed with paraformaldehyde and stained with crystal violet to allow for colony formation quantification. The results are shown in Figure 14. As can be seen from Figure 14A, showing a picture of wells containing viable cells, i.e., forming colonies. Figure 14B shows the results in a bar graph, with the number of colony forming units (CFUs) for each treatment. As can be seen, cell proliferation is higher for the untreated cells and cells treated with irrelevant RNA, whereas cells treated with NE-SS9 (NE3) encapsulating the target siRNA showed lower cell proliferation, indicating an effect of the siRNA delivered on its target.

### Example 5: Enhanced RNA encapsulation with combination of (a) and (b)

The RNA encapsulation of NEs as described herein comprising both an ionizable cationic lipid (a) and a permanently cationic lipid (b) (exemplified by NE1 as obtained by example 1) was compared to the RNA encapsulation of NEs without the permanently cationic lipid (exemplified by NE1 obtained in the same manner as described for NE1 in example 1 but without the permanently cationic lipid; the amount of permanently cationic lipid was replaced by the ionizable cationic lipid C12.

The results are shown in Figure 15, wherein the % of RNA encapsulation is significantly increased when using the combination of ionizable lipid (a) and permanently cationic lipid (b) in NE1 compared to the use of the ionizable lipid only in NE1, added in additional amount to exactly replace the mol% of the cationic lipid.

RNA encapsulation, was determined using Quant-iT RiboGreen RNA assay kit (Invitrogen), according to the manufacturer's instructions.

### Example 6: pKa adjustment by varying ratio of ionizable:cationic lipid

To evaluate the effect of the lipid combination ionizable cationic lipid (a) and a permanently cationic lipid (b) on the apparent pKa of the NEs, an assay was performed as follows.

The apparent pKa of the NEs was measured via the TNS (6-(p-Toluidino)-2-naphthalenesulfonic acid) assay. NEs were diluted in 0.9% (w/v) NaCl to a final concentration of 1 µg/mL and incubated for 5 min in a series of buffers containing 20 mM citrate, MES, HEPES, and boric acid in 0.9% NaCl, with pH values ranging from pH 3.0 to 11.0 and a final TNS concentration of 16 µM. The mean fluorescence intensity was measured in a black-bottom 96-well plate (Synergy H1 microplate reader, BioTek, VT, USA) at λEx = 321 nm and λEm = 447 nm at 37°C. The apparent pKa was defined by the pH at half-maximum fluorescence. Variants of NE1 (as prepared according to Example 1) were evaluated, where the total amount of lipid (a) and (b), in this composition equivalent to a total of 21.1 mol%, was split in a relative ratio (a):(b) of 44:1. The results are shown in Figure 16, wherein a significant increase in apparent pKa of the NE1-variants was observed with increasing amount of cationic lipid (b). In particular NEs without lipid combination (i.e., with a (a):(b) ratio of 100:0) have an apparent pKa around 6.0, and the apparent pKa lying between 6.2 and 6.7 for NEs with increasing amounts of (b). Without being bound to any theory, such apparent pKas may contribute to ensure that the droplets of the NEs are neutral in circulation through the body and that they are positively charged within the acidifying endosomes, improving tolerability and efficacy of NEs for the delivery of their cargo. Additionally, the ability to adjust the apparent pKa of the NEs by varying ratios of (a):(b) might cell specific delivery.

### Example 7: Enhanced in vitro transfection efficiency due to combination (a) and (b)

NE delivery was evaluated *in vitro* in HeLa cells that stably express GFP (HeLa-GFP) at a concentration 50 nM siRNA-GFP using the two variants of NE1 described in Example 5: NE1 with the lipid combination (a) and (b) and without the combination (including lipid (a) only). The expression of GFP upon treatment with these NEs was compared to the expression levels of GFP in untreated cells. The results show that the combination of (a) and (b) of NEs of the invention (with the combination (a) and (b) as exemplified by NE1) enhanced the delivery efficiency (Figure 17A) compared to NE1 without (b) as evidenced by a reduction in the expression of GFP in HeLa-GFP. On the other hand, the cell viability of the treated cells was not affected when compared to the untreated cells (Figure 17B).

### Example 8: Enhanced in vitro transfection efficiency due to surfactant

NE delivery was evaluated *in vitro* in HeLa cells that stably express GFP (HeLa-GFP) at a concentration 10 nM siRNA GFP comparing NE1 prepared according to example 1 (with the surfactant T80 in combination with a PEG-lipid) with a variation of NE1 that contains the PEG-lipid alone (by replacing the 7 mol% of non-ionic surfactant (T80) in the formulation NE1 by a 7mol% of additional Vit. E amount). The expression of GFP upon treatment with these NEs (10 nM) was compared to the expression levels in untreated cells (0 nM) as shown in Figure 18. The results show that the presence of the surfactant in NEs of the invention (as exemplified by NE1) enhanced the delivery efficiency compared to NE1 without the surfactant (T80) as evidenced by a reduction in the expression of GFP in HeLa-GFP.

## Claims

1. **An oil-in-water** nano-emulsion composition comprising:
(a) an ionizable cationic lipid,
(b) a permanently cationic lipid,
(c) an oil,
(d) a surfactant,
(e) an active ingredient comprising an oligonucleotide and/or a polynucleotide,
(f) an aqueous medium,
optionally comprising at least one of
(g) a PEG-lipid,
(h) an amphiphilic polymer, and
(i) a hydrophilic polymer,
and further optionally comprising
(j) an additional lipid selected from
∘ a phospholipid,
∘ a fatty acid,
∘ a fatty acid derivative, and
∘ a fatty amine,
wherein the nano-emulsion composition comprises
∘ from 8.0 to 60.0 mol% of ionizable cationic lipid (a),
∘ from 8.0 to 40.0 mol% of permanently cationic lipid (b), and
∘ a molar ratio of (a) to (b) from 1:1 to 3:1,
wherein the mol % is with respect to the total mols of (a), (b), (c), (d), and, if present, (g) and (j).

2. **The** composition of claim 1, comprising at least one of (g), (h) and (i), and preferably, wherein
- the PEGylated-lipid (g) is selected from 1,2-distearoyl-sn-glycero-3-phosphoethanolamine-poly(ethylene glycol) (DSPE-PEG), 1,2-dimyristoyl-sn-glycero-3-methoxypolyethylene glycol (DMG-PEG), and 1,2-distearoyl-rac-glycero-3-methylpolyoxyethylene (DSG-PEG);
- the amphiphilic polymer (h) is a partly negatively charged polymer, and more preferably is polyglutamic acid (PGA)-PEG; and/or
- the hydrophilic polymer (i) is a negatively charged hydrophilic polymer, and more preferably the hydrophilic polymer is selected from hyaluronic acid (HA), dextrane, polysialic acid (PSA) and alginate.

3. The composition of claim 1 or 2, wherein
- the ionizable cationic lipid (a) is selected from C12-200 (C12), 306Oi10 (306), SS-OP (SS9), 1,2-dioleoyl-3-dimethylammonium-propane (DODAP), D-Lin-MC3-DMA (MC3) or cKK-E12 (E12), is preferably selected from C12, 306 and SS9, more preferably is C12, 306 or SS9 and most preferably is 306 or SS9; and/or
- the permanently cationic lipid (b) is selected from 1,2-dioleoyl-3-trimethylammonium propane (DOTAP), DOTAP variants such as (N)-[1-(2,3-dioleyloxy)propyl]-N,N,N-trimethylammonium chloride) (DOTMA) and/or (1,2-distearoyl-3-trimethylammonium propane) (DSTAP), and cholesterol-based permanently cationic lipids such as (3β-[N-(N-N-dimethylaminoethane)-carbamoyl]cholesterol) (DC-Chol) and/or (Ethylphosphocholine-cholesterol) (EPC-Chol), preferably the the permanently cationic lipid (b) is DOTAP.

4. The composition of any one of claims 1 to 3, comprising the additional lipid (j), and preferably, wherein in the additional lipid (j)
- the phospholipid is selected from 1,2-dioleoyl-sn-glycero-3-phosphoethanolamine (DOPE), distearoylphosphatidylcholine (DSPC) or soy L-alpha-phosphatidylcholine (soy PC), and/or 1,2-dioleoyl-sn-glycero-3-phospho-(1'-rac-glycerol) (DOPG);
- the fatty acid is linoleic acid;
- the fatty acid derivative is glycerol-monooleate; and/or
- the fatty amine is oleylamine;
more preferably, the additional lipid (j) is a phospholipid, yet more preferably the additional lipid (j) is a phospholipid selected from DOPE, DSPC, and soy PC and even more preferably the additional lipid (j) is DOPE.

5. The composition of any one of claims 1 to 4, wherein the oil (c) is selected from Vitamin E (Vit E), oleic acid, ethyl oleate, isopropyl myristate, soybean oil or squalene and squalene derivatives, and a combination of medium-chain triglycerides (TGs), the combination of medium chain TGs preferably comprises caprylic acid and capric acid, and more preferably comprises Labrafac^{™} Lipophile, WL 1349 (WL 1349) or Labrafac^{™} PG (PG); and the oil preferably is Vit E.

6. The composition of any one of claims 1 to 5, wherein the surfactant (d) is a non-ionic surfactant, and preferably the non-ionic surfactant is selected from Tween^{®} 80 (T80), Kolliphor^{®} HS 15 (HS15), TPGS (α-Tocopherol polyethylene glycol succinate), and poloxamers, and more preferably the non-ionic surfactant is T80.

7. The composition of any one of claims 1 to 6, wherein
- the concentration of the oil (c) is from 20 to 79 mol%, and/or
- the concentration of the surfactant (d) is from 0.1 to 40 mol%,
wherein the mol% is with respect to the total mols of (a), (b), (c), (d), and, if present, (g) and (j).

8. The composition of any one of claims 1 to 7, wherein the active ingredient (e) is selected from therapeutic oligonucleotides and polynucleotides, more preferably is a therapeutic oligonucleotide, yet more preferably the therapeutic oligonucleotide is a single stranded (ss) and/or double stranded (ds) therapeutic oligonucleotide.

9. The composition of any one of claims 1 to 8, wherein the active ingredient (e) is selected from a RNA-based therapeutic nucleotide, preferably is selected from a messenger RNA (mRNA)-, a small interfering RNA (siRNA)-, a micro RNA (miRNA)-, a long noncoding RNA (IncRNA)-, a circular RNA (circRNA)-, an antisense oligonucleotide (ASO)-, self-amplifying RNA (saRNA)-, an activating RNA (aRNA)-, an editing RNA (eRNA)-, an inhibiting RNA (iRNA)-, a single-stranded guide RNA (sgRNA)-, a transfer RNA (tRNA)-, a decoy RNA-based therapeutic nucleotide and a combination thereof, and more preferably the active ingredient is selected from a mRNA and a siRNA.

10. The composition of any one of claims 1 to 11, wherein
- the active ingredient (e) is present at a concentration of at least 0.0125 mg/mL with respect to the total volume of the nano-emulsion, preferably from 0.0125 mg/mL to 10.0 mg/mL, and/or
- the weight ratio of active ingredient (e) to the total weight of (a), (b), (c), (d), and, if present, (g) and (j), is from 1:5 to 1:100.

11. The composition of any one of claims 1 to 10, wherein the oil phase is in the form of droplets dispersed in the aqueous phase having
(I) an average diameter of ≤ 300 nm, preferably of ≤ 100 nm, more preferably from 10 to 80 nm, and yet more preferably from 25 to 62 nm;
(II) a zeta potential from -30 mV to +35 mV, preferably -10 mV to +8 mV, more preferably from - 5 to + 5 mV, and yet more preferably from - 2 to + 2 mV;
(III) a polydispersity index (PDI) of below 0.35, preferably below 0.25; and/or
(IV) an encapsulation efficiency (EE) of at least 60 %, preferably at least 90% more preferably at least 95%, yet more preferably of 100%.

12. The composition of any one of claims 1 to 11, wherein the aqueous medium (f) is selected from water, saline aqueous solution, buffered aqueous solution or a saline buffered aqueous solution, and preferably the aqueous medium is a saline buffered aqueous solution selected from a phosphate buffer saline, a tris buffer saline, an acetate buffer saline, a bicarbonate buffer saline, a carbonate buffer saline, a citrate buffer saline, and a succinate buffer saline.

13. A lyophilized composition, wherein the composition is an oil-in-water nano-emulsion composition according to any one of claims 1 to 12.

14. The composition of any one of claims 1 to 12 or the lyophilized composition of claim 13 for use in the treatment or prevention of a disease or a disorder, preferably for the treatment or prevention of a CNS-associated diseases or disorders, PNS-associated diseases or disorders, autoimmune diseases or disorders, liver-associated diseases or disorders, lung-associated diseases or disorders, immunological diseases or disorders and cancers and infectious diseases or disorders.

15. A method for the preparation of the nano-emulsion composition of any one of claims 1 to 12 or the lyophilized composition of claim 13, the method comprising the steps of:
1) dissolving (a) an ionizable cationic lipid, (b) a permanently cationic lipid, (c) an oil, (d) a surfactant and, if present, (j) an additional lipid selected from a phospholipid, a fatty acid, a fatty acid derivative; and a fatty amine, in a water-miscible organic solvent, preferably in ethanol, to provide a water-miscible organic phase;
2) dissolving (e) an active ingredient in an aqueous medium (f) to provide an aqueous phase; and
3) mixing the water-miscible organic phase and the aqueous phase to provide the nano-emulsion, and, when the method is for the preparation of the lyophilized composition, the method further comprising:
4) lyophilizing the nano-emulsion provided in step 3).

16. A method of treatment or prevention of a disease or a disorder, preferably for the treatment of a CNS-associated diseases or disorders, PNS-associated diseases or disorders, autoimmune diseases or disorders, liver-associated diseases or disorders, lung-associated diseases or disorders, immunological disorders and cancer, the method comprising administering the nano-emulsion composition of any one of claims 1 to 12 to a subject suffering from the disease or disorder, to a healthy subject or to a subject susceptible of contracting the disease or disorder.
